# EUROPEAN PATENT APPLICATION

(11) **EP 4 470 558 A1**
(43) Date of publication of application: **04.12.2024**
(21) Application number: 23176569.4
(22) Date of filing: 31.05.2023
(51) Int. Cl.: A61K 39/12, A61P 31/14, C07K 14/005, C12N 15/86

(54) **CORONAVIRUS DERIVED POLYNUCLEOTIDES AND THEIR USE AS VACCINES**

(71) Applicant: Consejo Superior de Investigaciones Cientificas, 28006 Madrid (ES)
(72) Inventor: ENJUANES SÁNCHEZ, Luis, 28049 Madrid (ES); SOLA GURPEGUI, Isabel, 28049 Madrid (ES); ZÚÑIGA LUCAS, Sonia, 28049 Madrid (ES); HONRUBIA BELENGUER, José Manuel, 28049 Madrid (ES); LORETO BELLÓ-PÉREZ, Melissa, 28049 Madrid (ES); MUÑOZ SANTOS, Diego, 28049 Madrid (ES)
(74) Representative: Uexküll & Stolberg

(57) **Abstract**

The present invention provides polynucleotides comprising:
(a) a polynucleotide sequence of SEQ ID NO: 43 (SARS-CoV-2Δ[3, E, 7 and 8] mutant) or SEQ ID NO: 42 (SARS-CoV-2Δ[3, E, 6 and 7] mutant); or
(b) a variant of SEQ ID NO: 43 or a variant of SEQ ID NO: 42, wherein the variant has at least 96 % identity, preferably at least 97 % or at least 98 % or at least 99 % identity to SEQ ID NO: 43 or SEQ ID NO: 42.

The invention further provides expression constructs, VLPs, cells and vaccines comprising the VLPs or polynucleotide as well as the use thereof for inducing protection against against SARS-CoV-2 infection.

## Description

### FIELD OF THE INVENTION

The present invention relates to polynucleotides comprising sequences encoding mutants of the SARS-CoV-2 RNA genome that are replication-competent and propagation-defective. The invention further relates to Virus Like Particles (VLPs), host cells and expression vectors comprising respective polynucleotides. The invention also relates to vaccine compositions comprising respective polynucleotides and their use for protecting a patient against SARS-CoV-2 infection.

### TECHNICAL BACKGROUND

Coronaviruses (CoVs) are a family of single-stranded positive polarity RNA (ssRNA+) viruses that have a large RNA virus genome, ranging in length from approximately 25 to 33 kilobases (kb). During a coronavirus infection, replication of genomic RNA (gRNA) and synthesis of a set of subgenomic RNAs (sgRNAs) of positive and negative polarity occurs without retrotranscription.

Coronaviruses mainly infect birds and mammals, and humans as well. The outbreak of Severe Acute Respiratory Syndrome (SARS) in 2002 and, more recently, Middle East Respiratory Syndrome (MERS) in 2012 have demonstrated the lethality of CoVs when they cross the species barrier and infect humans. In 2019, the SARS-CoV-2 outbreak in China has triggered a worldwide pandemic with much higher economic and health consequences than those caused by SARS-CoV and MERS-CoV. Vaccines against these pathogens are therefore needed and RNA replicon-based vaccines are one option.

Propagation-deficient RNA replicons are excellent platforms for vaccine generation, as they are a subtype of virus-derived vaccines, with a single infectious cycle that cannot spread from cell to cell. The deficiency in one or more essential functions (viral particle assembly and/or dissemination) makes them very safe vaccines and highly useful vectors for immunisation against infectious agents. To amplify these replicons, it is desirable to complement in trans the viral genes required for their propagation which have been previously removed. To do this, replicons can be grown in cell lines that complement and express the proteins required for their dissemination, which they lack, or by providing the missing proteins in trans using expression vectors. When replicons are grown in cells that do not complement their deficiencies, for example within the subject that has been vaccinated with that RNA replicon, they express their deficient genomes and the antigens they encode, without being able to produce infectious virions that propagate from cell to cell.

The present invention relates to replication-competent but propagation-defective RNA replicons. Some of the advantages of using RNA replicons as platforms for vaccine generation are: (i) their easy intranasal administration, (ii) they have only one infection cycle due to the deleted genes, (iii) they do not integrate into the genome since they are RNAs, (iv) their biosafety; and (v) self-replication inside the cell and expression of high levels of viral antigens.

The arrangement of genes in the coronavirus genome is: 5'-UTR (untranslated region) - replicase/transcriptase - spike (S) protein - envelope (E) protein - membrane (M) protein - nucleocapsid (N) protein - 3' UTR end and poly (A) tail. All four structural proteins (S, E, M and N) contribute to the efficient formation of structurally stable viral particles.

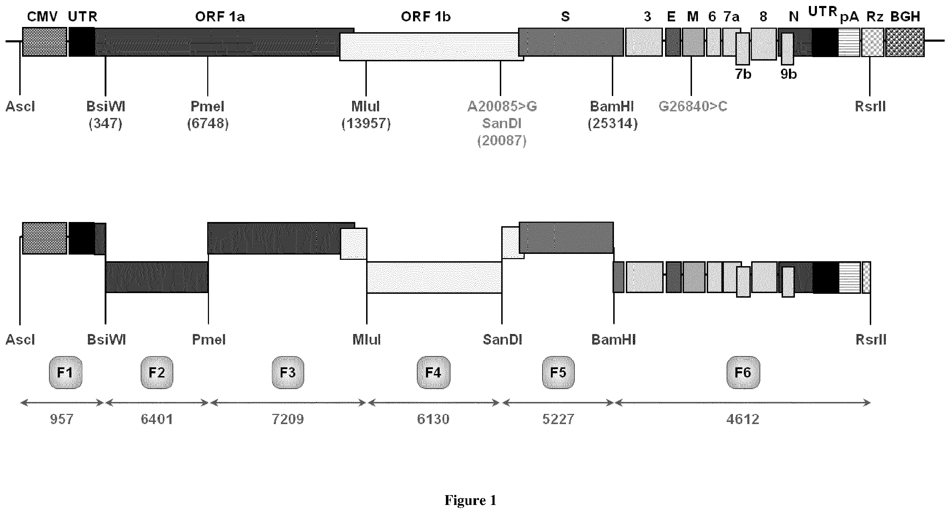

In addition to the structural genes, the coronavirus genome contains genes encoding proteins with non-structural functions, e.g. RNA replicase/transcriptase. Other genes that do not encode structural proteins are in the genome downstream of the replicase/transcriptase gene. Some genes encoding genus-specific accessory proteins are involved in counteracting host defences. Coronavirus genes are referred to as ORF (Open Reading Frame) followed by a number. The following table describes the distribution of genes in the SARS-CoV-2 genome (**Table 1**) The arrangement of the genes within the viral genome is illustrated in **Figure 1****.** The start and end nucleotides refer to the coding sequences, they do not include regulatory parts.

**Table 1. Genes present and their distribution in the SARS-CoV-2 genome**

| ORF | PROTEIN | START (nt) | END (nt) |
|---|---|---|---|
| 1ab | pp1ab | 266 | 21555 |
| 2 | S | 21563 | 25384 |
| 3a | 3 | 25393 | 26220 |
| 3b | - | 25814 | 25882 |
| 4 | E | 26245 | 26472 |
| 5 | M | 26523 | 27191 |
| 6 | 6 | 27202 | 27387 |
| 7a | 7a | 27394 | 27759 |
| 7b | 7b | 27756 | 27887 |
| 8 | 8 | 27894 | 28259 |
| 9 | N | 28274 | 29533 |
| 9b | 9b | 28305 | 28577 |

| | | | |
|---|---|---|---|
| (nt) stands for nucleotide | | | |

ORFs 1a and 1b encode the viral replicase, which is auto-proteolyzed leading to up to 16 non-structural proteins (nsps) encoding all functions required for viral replication (polymerase, helicase, primase, etc), RNA metabolism (exonuclease, endonuclease), viral mRNAs capping (methyltransferases) and other functions needed for the formation of membrane structures where viral replication takes place. In addition, many nsps are also involved in the modulation of the host innate immune response.

ORFs 2, 4, 5 and 9 encode the structural proteins spike (S), envelope (E), membrane (M) and nucleocapsid (N), respectively. S protein is involved in SARS-CoV-2 interaction with the host cell receptor (ACE-2) and determines viral tropism. M, E, and N proteins have an essential role in virus assembly and egress. E protein is a virulence factor. N protein interacts with the RNA viral genome to form the nucleocapsid, and this protein also has multiple functions in virus-host interaction.

It has also been described that ORFs 3ab, 6, 7ab, 8 and 9b encode accessory genus-specific proteins. Some of these proteins are involved in counteracting hosts defenses.

WO2018160977 discloses an attenuated coronavirus by an alteration in the replicase gene. The present invention is an improvement in that, by introducing changes, like the one described below on the nsp1, numerous antigens are produced, thus increasing the efficacy of the vaccine.

The scientific article Zhang et al. 2021. Cell discloses a replicon competent in replication, but defective in propagation, however, the replicon of the present invention is safer due to a series of additional deletions. Additionally, the E gene is completely deleted, whereas in the replicons of the present invention the gene E is partially deleted so that it does not affect the transcription of the M gene.

The scientific article Silvas et al. 2021. J Virol discloses the effect on pathogenicity produced by deletion of the SARS-CoV-2 ORFs individually creating attenuated viruses. This document does not disclose an RNA replicon of this virus, nor does it disclose the effect on immunogenicity of deleting these ORFs individually or combinations thereof. Additionally, it does not disclose with enough detail how the mutants were engineered. Therefore, a third party could not reproduce the viruses disclosed.

PCT/EP2022/083289 discloses other replication-competent and propagation-defective polynucleotides as well as the use thereof for protecting a patient against SARS-CoV-2 and other coronavirus infections. The methods disclosed in PCT/EP2022/083289 for cloning the SARS-CoV-2 genome and for generating mutants of the SARS-CoV-2 genome have also been used to generate the mutants of the present invention.

As illustrated by PCT/EP2022/083289 there is still a need for advantageous SARS-CoV-2 polynucleotides which can be used as vaccines for inducing protection against subsequent SARS-CoV-2 infection.

### SUMMARY OF THE INVENTION

For solving the problem outlined above, the present invention provides polynucleotides comprising:
(a) a polynucleotide sequence of SEQ ID NO: 43 (SARS-CoV-2Δ[3, E, 7 and 8] mutant) or SEQ ID NO: 42 (SARS-CoV-2Δ[3, E, 6 and 7] mutant); or
(b) a variant of SEQ ID NO: 43 or a variant of SEQ ID NO: 42, wherein the variant has at least 96 % identity, preferably at least 97 %, at least 98 % or at least 99 % identity to SEQ ID NO: 43 or SEQ ID NO: 42.

The present invention further provides polynucleotide as described above, wherein the variant polynucleotide sequence is further characterized in that:
(b1) the variant encodes a replication-competent mutant of the SARS-CoV-2 RNA genome, wherein replication-competent means that the mutant SARS-CoV-2 RNA genome can produce copies upon transfection of VeroE6-TMPRSS2 cells; and
(b2) the variant encodes a propagation-defective mutant of the SARS-CoV-2 RNA genome, wherein propagation-defective means that upon transfection of VeroE6-TMPRSS2 cells the mutant SARS-CoV-2 RNA genome is not able to assemble a virus or a VLP on the basis of the proteins encoded in the variant polynucleotide, which virus or VLP would be capable of infecting other VeroE6-TMPRSS2 cells.

In a related embodiment, the present invention provides polynucleotide as described above, wherein the variant polynucleotide sequence is further characterized in that:
(c) upon infection or transfection of a VeroE6-TMPRSS2 cell culture the mutant SARS-CoV-2 RNA genome is able to produce a viral titre of at least 10³ FFU/ml or a viral titre of at least 10⁴ FFU/ml or at least 10⁵ FFU/ml or at least 10⁶ FFU/ml.

In a preferred embodiment the polynucleotide as described above is RNA or DNA.

The present invention provides Virus Like Particles (or VLPs) which comprise the polynucleotides as described above.

The invention also provides expression vectors comprising a DNA sequence comprising polynucleotides of the present invention as described above.

In a further embodiment the invention provides cells transfected with a polynucleotide according the present invention or infected with a VLP of the present invention.

The VLPs described above can be made by any method for producing respective VLPs. In one alternative the methods for producing respective VLPs comprise the steps of:
(a) transfecting a cell with an expression vector as described above, wherein the cell expresses proteins encoded in SEQ ID NO: 6 and SEQ ID NO: 7 amino acid residues sequences; and
(b) purifying the VLPs from the supernatant.

In a separate embodiment, the present invention provides vaccine compositions comprising a polynucleotide as described above or a VLP as described above, and optionally further comprising:
- at least one pharmaceutically acceptable excipient and/or
- at least one chemical or biological adjuvant or immunostimulant.

The vaccine compositions of the present invention can be used for protecting a patient against subsequent SARS-CoV-2 infection.

### DETAILED DISCLOSURE

### Definitions

The terms used to characterize the present invention are to be interpreted according to their common meaning in the art, except for the terms explicitly defined herein.

In accordance with the present invention, percent sequence identity between two polynucleotides is determined using the publicly available EMBOSS Supermatcher software using the standard settings (https://www.bioinformatics.nl/cgi-bin/emboss/supermatcher), which provides a combination of a word-match and Smith-Waterman local alignment algorithms.

The present invention further provides polynucleotides that are further characterized as encoding a replication-competent mutant of the SARS-CoV-2 RNA genome. The term "replication-competent" is understood as meaning that the polynucleotide can produce copies of itself upon transfection of VeroE6-TMPRSS2 cells.

The present invention further provides polynucleotides that are further characterized as encoding a propagation-defective mutant of the SARS-CoV-2 RNA genome. The term "propagation-defective" is understood as meaning that upon transfection of VeroE6-TMPRSS2 cells the polynucleotide is not able to assemble a virus or a VLP on the basis of the proteins encoded in the polynucleotide, which virus or VLP would be capable of infecting other VeroE6-TMPRSS2 cells.

In the present specification, when "deletion of a gene" is indicated, it may be a total or partial deletion of a nucleotide sequence when the exact alteration is not indicated. It can be a deletion of any nucleotide length or also be several deletions along the nucleotide sequence, it can be any type of deletion as long as the coding protein is not functional.

According to the present invention, a "bacterial artificial chromosome" (BAC) is a DNA sequence comprising the F-factor sequence. Plasmids containing this sequence, called F-plasmids, can stably maintain heterologous sequences of a length greater than 300 kb with a maximum of one or two copies per cell. The corresponding BACs can be any known in the state of the art.

The term "virus-like particles" (or VLPs) is used to characterize a particle comprising a polynucleotide and associated proteins, both derived from SARS-CoV-2 sequences. The polynucleotide is preferably an RNA polynucleotide that encodes a replication-competent and propagation-deficient deletion mutant of SARS-CoV-2. The VLP further comprises structural proteins required for viral assembly and for infection of additional cells. Insofar as these structural genes were deleted from the propagation-deficient polynucleotides of the present invention, the VLPs of the invention are obtained using cell lines, containing the viral genes as part of the genome of the cell, which cell lines are capable of complementing these proteins.

SARS-CoV and SARS-CoV-1 are synonymous and both terms refer to the first SARS-CoV that emerged in 2002.

The term "coronavirus" is used according to the present invention to refer to a group (Family) of viruses having a single molecule of linear, positive-sense, single stranded ssRNA of 25 to 33 kb. The term coronavirus includes any member of the family *Coronaviridae*, preferably *Orthocoronaviridae*, and more preferably of the genus *Betacoronavirus* and even more preferably SARS-CoV-2.

In the present specification "genes encoding genus-specific accessory proteins" are those genes in the coronavirus genome that encode the synthesis of proteins that are most frequently not incorporated into the virus structure.

In the present specification "expression vector" can be a bacterial artificial chromosome (BAC), a cosmid and/or a P1-derived artificial chromosome.

The term "polynucleotide" or "nucleic acid" as used in this description refers to nucleotide sequences consisting of at least 100 nucleotides and includes genes or gene fragments, as well as, in general, any DNA or RNA molecule, single or double stranded.

In the present specification, the term "replicon" is synonymous with "RNA replicon" and refers to an RNA polynucleotide that is replication-competent as described above (since it can make many copies of itself), but defective in propagation as described above. The replicon is an RNA polynucleotide. Insofar as the present application characterizes the replicon by reference to a DNA polynucleotide sequence provided in the sequence listing it is to be understood that the replicon is in fact characterized by the corresponding RNA sequence. For example, a replicon comprising the sequence of SEQ ID NO: 2 is a replicon comprising an RNA sequence that corresponds to the DNA sequence of SEQ ID NO: 2.

The replicon of the present invention can form virus-like particles (VLPs) formed from subgenomic RNAs which act as messenger RNAs and are translated into proteins that assemble into structures giving rise to VLPs which wrap the RNA replicon. Proteins required to produce VLPs which are not encoded in the replicon must be provided in trans.

In the present specification, the expression "inducing protection", should be understood as inducing an immune response in the recipient organism, mediated by antigens generating a long-term memory effect therein, said antigen being encoded by the RNA replicon of the invention. This immune response may be enhanced by mechanisms involving the induction of substances that enhance the humoral response mediated by antibodies, or cellular, mediated by interleukins, cytokines, interferons, or the like, and substances that mediate intracellular processes that cause the subject to be protected against infections caused by infectious agents.

The term "vaccine" and "vaccine composition" are synonyms having the usual meaning in the field.

The expression "comprising the gene coding for..." means, in the present disclosure, that the polynucleotide sequence gives rise to a functional viral protein. Regardless of the fact that said sequence may have undergone alterations (point mutations, deletions or additions) with respect to the canonical sequence of said gene. Similarly, RNA replicons of the present invention which are characterized as not comprising "sequences suitable for expressing" a specified ORF protein are RNA polynucleotides which do not contain sequences that would provide for a respective protein to be expressed by the RNA polynucleotides in cell culture under conditions, where WT SARS-CoV-2 would express the corresponding ORF protein.

Coronavirus genes are named as ORF (open reading frame) plus a number. In the present specification, when a coronavirus gene is mentioned, the number can be or not preceded with ORF: i.e. ORF3, gene 3.

In the present specification the number "3" within the name of an RNA replicon or an attenuated virus comprises gene 3a and 3b. These 2 genes can also be referred as 3ab.

In the present specification the number "7" within the name of an RNA replicon or an attenuated virus comprises genes 7a and 7b. These 2 genes can also be referred as 7ab.

In the present specification rSARSCoV-2 and its derivatives, refers to the different recombinant viruses obtained from SARS-CoV-2 genome in the laboratory, they could be wild type (wt), attenuated viruses (with the deletions in genes that do not make the virus propagation deficient) or replicons.

### Polynucleotides Encoding SARS-CoV-2 Deletion Mutants

The present invention discloses replication-competent propagation-deficient polynucleotides derived from SARS-CoV-2 genome that have been constructed by the novel combination of specific sets of deleted genes. Based on an exhaustive analysis of the effect of deleting specific genes of this virus on its replication and virulence in humanized transgenic mice models, novel combinations of deleted genes were selected to analyse their attenuation and induction of protection. In particular, the present invention provides polynucleotides comprising sequences derived from the SARS-CoV-2 genome, which polynucleotides can be used for producing advantageous SARS-CoV-2 vaccines.

In a first aspect the invention provides polynucleotides comprising:
(a) a polynucleotide sequence of SEQ ID NO: 43 (SARS-CoV-2Δ[3, E, 7 and 8] mutant) or SEQ ID NO: 42 (SARS-CoV-2Δ[3, E, 6 and 7] mutant); or
(b) a variant of SEQ ID NO: 43 or a variant of SEQ ID NO: 42, wherein the variant has at least 96 % identity, preferably at least 97 %, at least 98 % or at least 99 % identity to SEQ ID NO: 43 or SEQ ID NO: 42.

In a preferred embodiment the invention provides polynucleotides comprising a variant of SEQ ID NO: 43 or a variant of SEQ ID NO: 42, wherein the variant has at least 99 % identity to SEQ ID NO: 43 or SEQ ID NO: 42.

In one aspect the polynucleotide of the invention can additionally be characterized in that:
(b1) the variant encodes a replication-competent mutant of the SARS-CoV-2 RNA genome, wherein replication-competent means that the mutant SARS-CoV-2 RNA genome can produce copies upon transfection of VeroE6-TMPRSS2 cells; and
(b2) the variant encodes a propagation-defective mutant of the SARS-CoV-2 RNA genome, wherein propagation-defective means that upon transfection of VeroE6-TMPRSS2 cells the mutant SARS-CoV-2 RNA genome is not able to assemble a virus or a VLP on the basis of the proteins encoded in the variant polynucleotide, which virus or VLP would be capable of infecting other VeroE6-TMPRSS2 cells.

In a related aspect, the polynucleotide can further be characterized in that:
(c) upon infection or transfection of a VeroE6-TMPRSS2 cell culture the mutant SARS-CoV-2 RNA genome is able to produce a viral titre of at least 10³ FFU/ml or a viral titre of at least 10⁴ FFU/ml (Focus Forming Units/ml) or at least 10⁵ FFU/ml or at least 10⁶ FFU/ml.

FFU (or Focus Forming Units) are determined using the focus forming assay, which is a immunoassay detecting infected host cells and infectious virus particles. The method comprises steps, wherein about 5 × 10⁴ VeroE6-TMPRSS2 cells are seeded per well in 96-well plates in 100 µL of media, the cells are infected with undiluted virus, and infected cells are detected after one day using immunofluorescence. Example 2.4 provides further details of one alternative of carrying out the method.

The present invention further provides polynucleotides comprising SEQ ID NO: 43 or SEQ ID NO: 42 or a variant of SEQ ID NO: 43 or SEQ ID NO: 42, which comprises a deletion of at least 18 consecutive nucleotides in the sequence encoding a full-length nsp1 domain consisting of nucleotides 266 to 805 of SEQ ID NO: 43 or SEQ ID NO: 42. The nsp1 sequence controls translation and deletion of amino acids in this region has been shown to be particularly advantageous for further attenuation and thus improved safety of the use of the polyncleotides as vaccines. Deleting consecutive polynucleotides from the nsp1 sequence is illustrated in Example 2.1, below.

The present invention also provides polynucleotides comprising SEQ ID NO: 43 or SEQ ID NO: 42 or a variant of SEQ ID NO: 43 or SEQ ID NO: 42, which comprises a deletion of at least 6 consecutive nucleotides in the sequence encoding a furin cleavage site consisting of nucleotides 23603 to 23617 of SEQ ID NO: 43 or SEQ ID NO: 42. Again, deleting parts of the furin cleavage site will significantly improve the safety of the use of the polyncleotides as vaccines.

The advantageous features of the polynucleotides can also be combined and in one aspect the invention provides polynucleotides comprising:
(a) a polynucleotide sequence of SEQ ID NO: 43 (SARS-CoV-2Δ[3, E, 7 and 8] mutant) or SEQ ID NO: 42 (SARS-CoV-2Δ[3, E, 6 and 7] mutant); or
(b) a variant of SEQ ID NO: 43 or a variant of SEQ ID NO: 42, wherein the variant has at least 98 % identity to SEQ ID NO: 43 or SEQ ID NO: 42, and
wherein the polynucleotide or the variant comprises a deletion of at least 18 consecutive nucleotides in the sequence encoding a full-length nsp1 domain consisting of nucleotides 266 to 805 of SEQ ID NO: 43 or SEQ ID NO: 42; and/or wherein the polynucleotide or the variant comprises a deletion of at least 6 consecutive nucleotides in the sequence encoding a furin cleavage site consisting of nucleotides 23603 to 23617 of SEQ ID NO: 43 or SEQ ID NO: 42.

The polynucleotide can be an RNA or a DNA polynucleotide and has a length of at least 100 nucleotides. The polynucleotide preferably has a length of less than 30 kb.

Methods for producing polynucleotides in general are well known in the art and can be used for producing the polynucleotides of the present invention. Respective methods are also illustrated in Examples 1 and may comprise steps of producing a full-length infectious cDNA clone of the SARS-CoV-2 viral RNA genome by dividing the cDNA corresponding to the SARS-CoV-2 genome into a number of fragments, chemically synthesizing the fragments and cloning the fragments stepwise into a bacterial artificial chromosome (BAC). In the BAC, the viral genomic cDNA can be flanked by a promoter, such as the CMV promoter. The viral genomic DNA can further be flanked by a ribozyme sequence, such as the hepatitis delta virus ribozyme sequence together with the bovine growth hormone polyadenylation and termination signals. These additional sequences will facilitate expression of the viral RNA from the cDNA BAC.

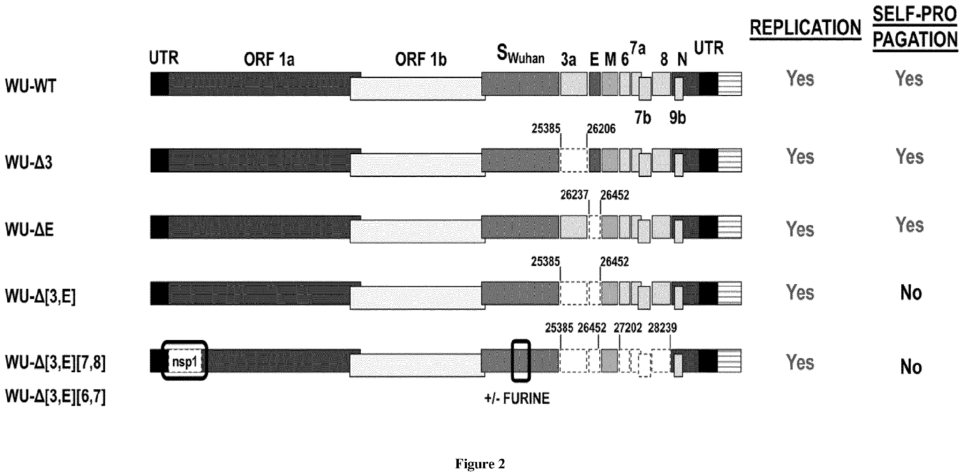

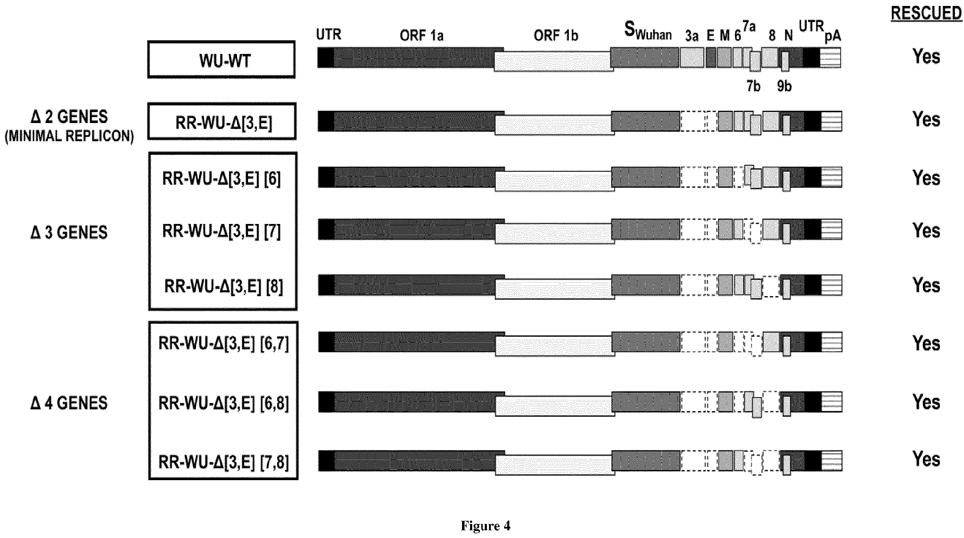

Based on the full-length infectious cDNA clone of the SARS-CoV-2, viral RNA genome deletion mutants can be produced. In a first step DNA fragments can be generated by chemical synthesis, which fragments contain SARS-CoV-2 polynucleotide sequences, wherein one or several viral genes were deleted (deletions are illustrated in Table 3, below; and in Figures 2 and 4). The fragments containing deletions are then inserted into the BAC sequence.

### Virus-like Particles

The present invention also provides virus-like particles comprising polynucleotides of the invention as described above.

In accordance with the disclosure of the present invention, the virus-like particles (VLPs) comprise a polynucleotide of the present invention as described above, preferably an RNA polynucleotide, as well as structural proteins required to assemble a virus and infect additional cells. Insofar as these structural genes were deleted from the polynucleotides of the present invention, the VLPs of the invention are produced in cell lines, changing the respective viral genes as part of the cellular genome and thus capable of complementation.

Methods of producing VLPs are well known in the art and illustrated in Example 8. These methods can be used to produce the VLPs of the present invention. These methods may for example comprise steps of:
(a) transfecting a cell with an expression vector as described above, wherein the cell expresses proteins encoded in SEQ ID NO: 6 and SEQ ID NO: 7 amino acid residues sequences; and
(b) purifying the VLPs from the supernatant.

### Expression Vectors

The present invention further provides expression vectors comprising a DNA sequence comprising a polynucleotide of the present invention. Any type of expression vector is encompassed by the invention, including plasmids and bacterial artificial chromosomes (BACs). The use of BACs for cloning SARS-CoV-2 viral genomic sequences is illustrated in Example 1. Numerous plasmids that can advantageously be used as expression vectors are known in the field and also illustrated in the Examples.

### Cells

The present invention provides cells comprising polynucleotides of the present invention and/or an expression vector of the present invention and/or a VLP of the present invention.

The host cell may be of prokaryotic or eukaryotic origin. The examples show that the initial cloning of the viral genome was carried out using BACs and *E. coli* cells.

The examples also illustrate infection and transfection of eukaryotic host cells, in particular using one of the following cell lines: Vero E6 cells, Vero E6-TMPRSS2 cells, BHK21 cells. It is well known that Vero cells do not express IFN and Vero cell lines are therefore well known and frequently used for maintaining viral sequences. These cell lines can be commercially obtained from numerous sources, including depository authorities. VeroE6-TMPRSS2 cells used in the Examples of the present application were obtained from the Centre For AIDS Reagents (National Institute for Biological Standards and Control, United Kingdom).

Insofar as a host cell is used for the production of VLPs using a polynucleotide encoding a SARS-CoV-2 deletion mutant of the present invention, the genome of the host cell comprises the sequences deleted from the viral genome so that the host cell is capable of complementing the structural proteins in the sense of providing the same for assembly of VLPs.

### Vaccines

The present invention provides vaccine compositions comprising a polynucleotide of the present invention or a VLP of the present invention. The composition may further comprise pharmaceutically acceptable excipients and/or chemical or biological adjuvants or stimulants. Respective excipients, adjuvants and immunostimulants are well known in the art and can be used for this purpose.

In a preferred embodiment the vaccine composition comprises a VLP of the present invention.

In one aspect, the invention provides vaccines comprising a VLP comprising a polynucleotide variant of SEQ ID NO: 43 or a variant of SEQ ID NO: 42, wherein the variant has at least 99 % identity to SEQ ID NO: 43 or SEQ ID NO: 42.

In a related aspect, the invention provides vaccines, comprising a VLP comprising a polynucleotide comprising:
(a) a polynucleotide sequence of SEQ ID NO: 43 (SARS-CoV-2Δ[3, E, 7 and 8] mutant) or SEQ ID NO: 42 (SARS-CoV-2Δ[3, E, 6 and 7] mutant); or
(b) a variant of SEQ ID NO: 43 or a variant of SEQ ID NO: 42, wherein the variant has at least 98 % identity to SEQ ID NO: 43 or SEQ ID NO: 42, and
wherein the polynucleotide or the variant comprises a deletion of at least 18 consecutive nucleotides in the sequence encoding a full-length nsp1 domain consisting of nucleotides 266 to 805 of SEQ ID NO: 43 or SEQ ID NO: 42; and/or wherein the polynucleotide or the variant comprises a deletion of at least 6 consecutive nucleotides in the sequence encoding a furin cleavage site consisting of nucleotides 23603 to 23617 of SEQ ID NO: 43 or SEQ ID NO: 42.

The vaccines can be used for protecting a patient, preferably a human patient, against subsequent SARS-CoV-2 infection. The vaccine can be formulated for topical, intranasal, oral, subcutaneous, or intramuscular administration.

The vaccine composition can be administered repeatedly. According to one aspect, the vaccine composition is administered at least two times in an interval of at least three weeks.

### Brief Description of the Figures

- **Figure 1:**: **SARS-CoV-2 genome scheme and fragments used for producing polynucleotides of the invention.** The upper panel scheme represents SARS-CoV-2 genome, wherein the letters above the boxes indicate viral genes: S, spike protein coding gene; E, envelope protein coding gene; M, membrane protein coding gene; N, nucleocapsid protein coding gene. The numbers, with or without letters, above the boxes indicate genus-specific genes. The construct is flanked by cytomegalovirus promoter (CMV) at the 5' end, and hepatitis delta virus ribozyme (Rz) plus bovine growth hormone polyadenylation and termination sequences (BGH) at the 3' end. pA, poly A tail. Grey letters indicate the silent mutations engineered as genetic markers: A20085>G, generating a unique SanDI restriction site, and G26840>C, eliminating Mlul and BsiWI restriction sites. Black letters indicate the unique restriction sites used for genome fragment assembly, their position in the viral genome is in brackets. pBAC-SARS-CoV-2 sequence is described in SEQ ID NO: 8.
The scheme in the lower panel represents the six fragments (F1 to F6) designed to engineer SARS-CoV-2 cDNA, flanked by the restriction sites selected for the assembly. Fragment size, in nucleotides (nt), is indicated below the arrows.
- **Figure 2:**: **Structural overview over SARS-CoV-2 human coronavirus genome and polynucleotides encoding SARS-CoV-2 deletion mutants.** Representation of the genome of SARS-CoV-2 strain Wuhan-Hu-1 (WU-WT, GenBank MN908947, SEQ ID NO: 1) and of the constructed SARS-CoV-2 deletion mutants (WU-Δ[3], WU-Δ[E], WU-Δ[3,E], WU-Δ[3,E,7,8], WU-Δ[3,E,6,7]). The letters above the boxes indicate viral genes: S_{Wuhan}, spike protein coding gene of strain Wuhan-Hu-1; E, envelope protein coding gene; M, membrane protein coding gene; N, nucleocapsid protein coding gene. The numbers, with or without letters, above the boxes indicate the genus-specific genes 3a, 6, 7a, 7b, 8, 9b. UTR, untranslated region; ORF, open reading frame; An, poly A tail. The last construct contains two encircled areas highlighting the domain of the nsp1 protein coding sequence, which can be modified by deletion of nucleotides, and the furin cleavage site which can also be modified by deletions in addition to the deletions introduced into the constructs comprising Δ[3,E,7,8] and Δ[3,E,6,7] polynucleotides. Genes 3a and E, and selected from the genes 6, 7a, 7b, and 8. Numbers above the boxes indicate the nucleotide position in the genome. The right panel provides information on the replication and self-propagation competence of the genome/replicon.

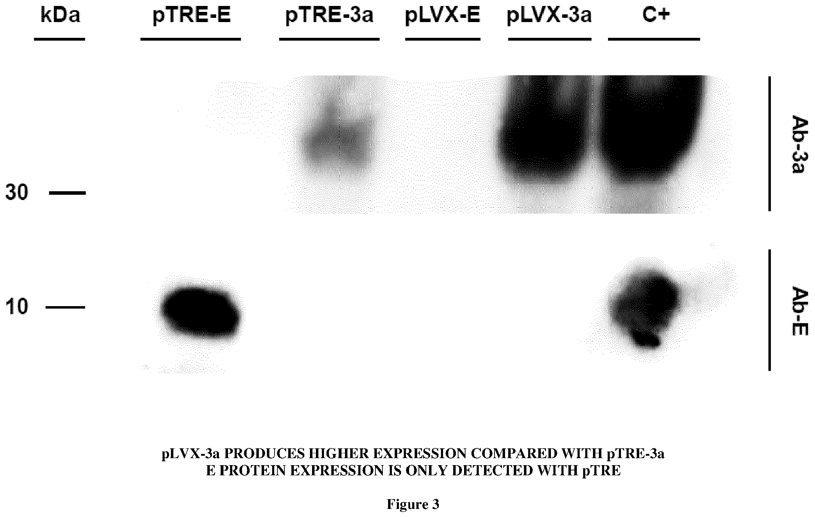

- **Figure 3:**: **Expression of SARS-CoV-2 3a and E proteins encoded by pTRE-E, pTRE-3a, pLVX-E and pLVX-3a plasmid (Example 2.3).** Detection of SARS-CoV-2 3a and E proteins by Western Blot analysis. Cell lysates were resolved by SDS-PAGE (*NuPAGE^{™} 4-12% Bis-Tris Gel, Invitrogen by Thermo Fisher Scientific, USA)* and transferred to PVDF membranes *(Immun-Blot PVDF Membranes for Protein Blotting, BIO-RAD, USA)* with a Mini Gel Tank electroblotting apparatus (*Invitrogen by Thermo Fisher Scientific, USA)* at 80V for two hours. Membranes were blocked for an hour at room temperature with 3% dried skimmed milk in T-TBS (TBS + 0.1% Tween 20) and then probed with the following primary antibodies: rabbit anti-E bleeding (diluted 1:500) and sheep anti-3a bleeding (diluted 1:1000) overnight at 4°C in T-TBS containing 1% dried skimmed milk. Bound primary antibodies were detected with horseradish peroxidase conjugated goat anti-rabbit IgG (Ref. *A0545, Sigma-Aldrich, Madrid, Spain)* and rabbit anti-sheep *(Ref. 31480, Invitrogen by Thermo Fisher Scientific, USA)* diluted 1:10000 in T-TBS with 1% dried skimmed milk, followed by incubation with the Clarity^{™} Western ECL Substrate *(BIO-RAD, USA)*, according to the manufacturer's recommendations.
- **Figure 4:**: **Structural overview over SARS-CoV-2 and polynucleotides encoding SARS-CoV-2 deletion mutants with two, three or four deleted genes (produced in Example 2).** The dashed areas represent the deleted genes from SARS-CoV-2 WU-WT to obtain the deletion mutants. The right panel indicates that all deletion mutants can be rescued. RNA replicons missing genes 3a and E are replication-competent, propagation-defective, and those in which two additional genes have been deleted among the three genes 6, 7a-7b or 8 are, in addition, attenuated in relation to the wild type RNA replicon. Deletion mutants were engineered using the SARS-CoV-2 cDNA clone from our lab. Viral genes are indicated at the top of the figure. White boxes within discontinuous lines represent deleted genes. The sequence of SARS-CoV-2 parental virus (wt) and the respective deletion mutants in which two genes 3 & E, (Δ[3,E]), or four genes: 3,E,7,8 (Δ[3,E,7,8] or Δ[3,E,6,7]) have been deleted, is shown. Additionally, nsp1 protein and furin cleavage site in the Spike protein can be eliminated to increase safety in Δ[3,E,7,8], Δ[3,E,6,8] & Δ[3,E,6,7] mutants. Viability shown on the right side of the Figure.

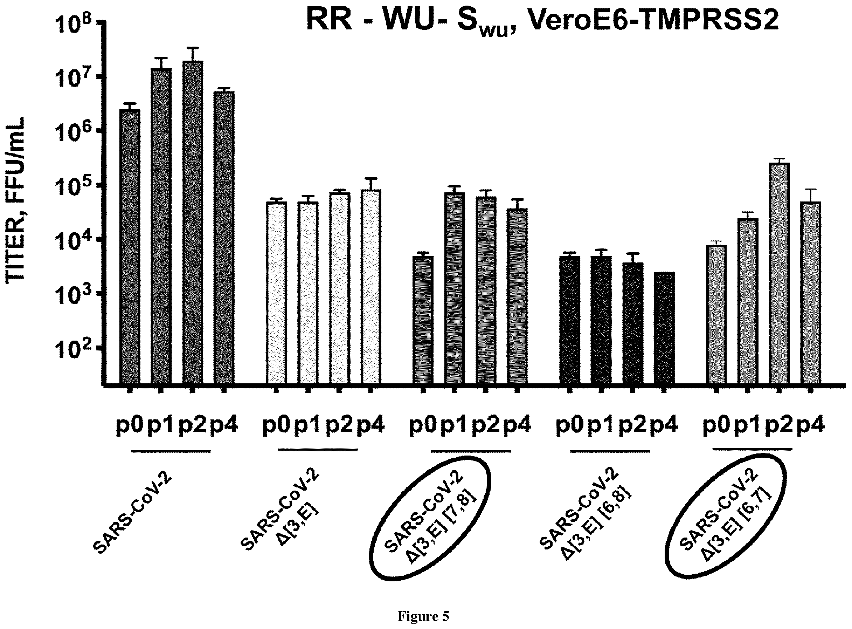

- **Figure 5:**: **Rescue and stability of SARS-CoV-2, SARS-CoV-2 Δ[3,E], SARS-CoV-2Δ[3,E,7,8], SARS-CoV-2Δ[3,E,6,8], SARS-CoV-2Δ[3,E,6,7] in VeroE6-TMPRSS2 cells.** Virus titers for the passage numbers p0, p1, p2, p4 are shown. RNA replicons in which four genes were deleted, that provided the highest titers were selected for further characterization (SARS-CoV-2 Δ[3,E,7,8] and SARS-CoV-2 Δ[3,E,6 7] based on their highest virus titers and higher number of deleted genes.

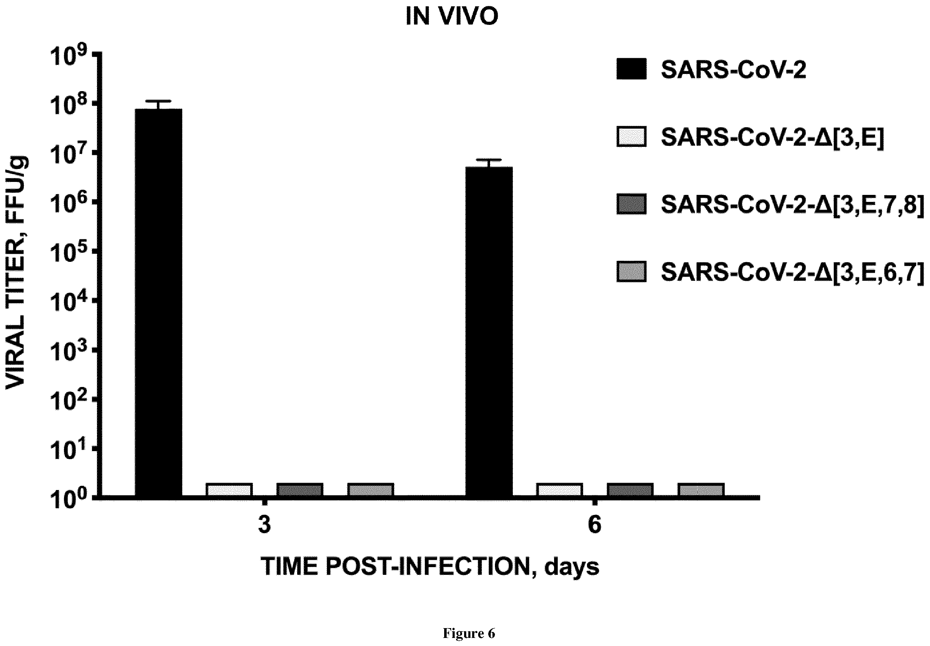

- **Figure 6:**: **The SARS-CoV-2Δ[3,E], SARS-CoV-2Δ[3,E,7,8] and SARS-CoV-2Δ[3,E,6,7] RNA replicons are propagation-deficient in mice lungs (Example 5).** Six 21-week-old female K18-hACE2 transgenic mice were intranasally inoculated with 10⁴ FFU/animal of each RNA replicon. Lung samples were obtained at 3- and 6-days post-infection and viral titers were determined (n = 3 mice per time point). The figure shows that in the absence of complementation by providing the deleted proteins in trans, the RNA replicons were not detected at days 3 or 6 post infection. The values represent means of 9 mice. Error bars indicate SEM.

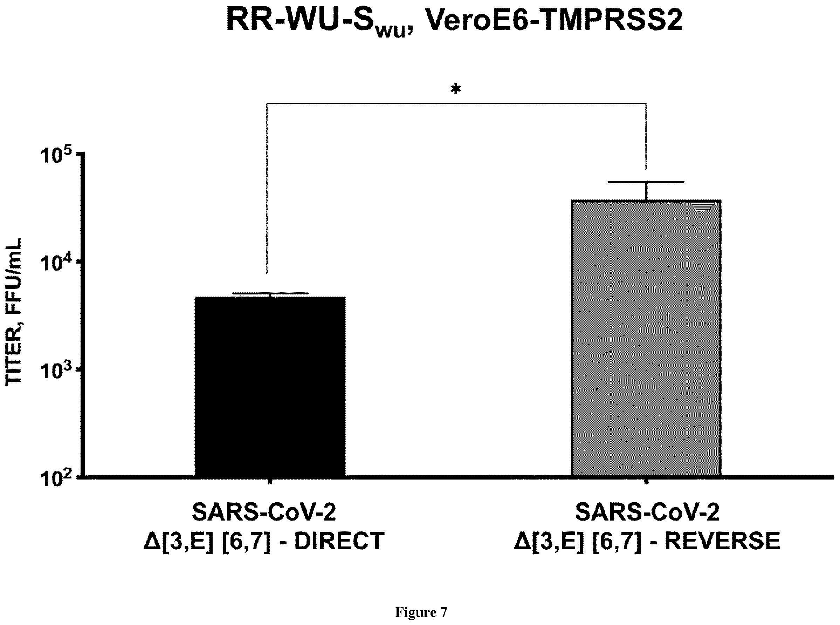

- **Figure 7:**: **Reverse transfection increases titers of the SARS-CoV-2Δ[3,E,6,7] RNA replicon in VeroE6-TMPRSS2 cells (Example 3).** Comparison of VLP titer in the rescue of the RR-WU-S_{wu}- Δ[3,E,6,7] mutant using direct or reverse transfection and providing 3a and E protein in trans. Supernatants were collected after 72 hours post transfection (hpt) and titrated by immunofluorescence technique as previously described (Gutierrez et al., 2021). Vertical bars represent the mean and the standard deviation of the data obtained in the lung of each of the three mice used for each experimental setting. Unpaired *t* test: *, *p* < 0.05. Results are represented as the mean ± SD. *, p < 0.05.

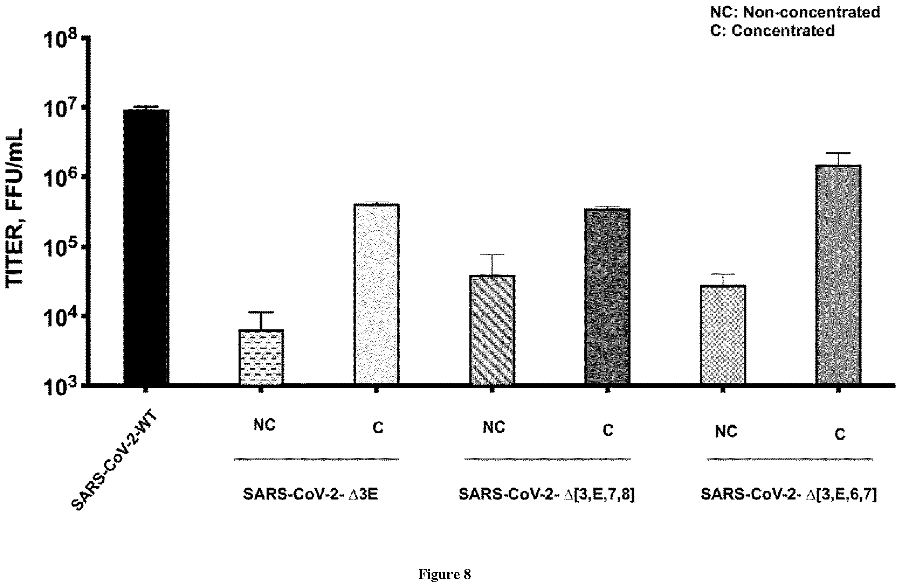

- **Figure 8:**: **Viral stocks of the SARS-CoV-2-Δ[3,E], SARS-CoV-2-Δ[3,E,7,8], SARS-CoV-2-Δ[3,E,6,7] RNA replicons in VeroE6-TMPRSS2 cells (Example 4).** Stock generation of RNA replicons-WU-S_{wu}-(Δ[3,E],-Δ[3,E,7,8] and -Δ[3,E,6,7]). Vero E6-TMPRSS2 were infected with each mutant at a MOI of 0.001, and 3a and E proteins were provided in trans. Supernatants were collected at 72 hpi and were non-concentrated (NC) or concentrated (C) using the *Intact Virus Precipitation Reagent (optimized for SARS-CoV-2)* (Invitrogen. Ref: 10720D). VLPs stocks were titrated by immunofluorescence technique. The vertical bars represent the mean and the standard deviation of the data. Results are represented as the mean ± SD. Figure 8 shows that high titer can be obtained by high expression or concentration

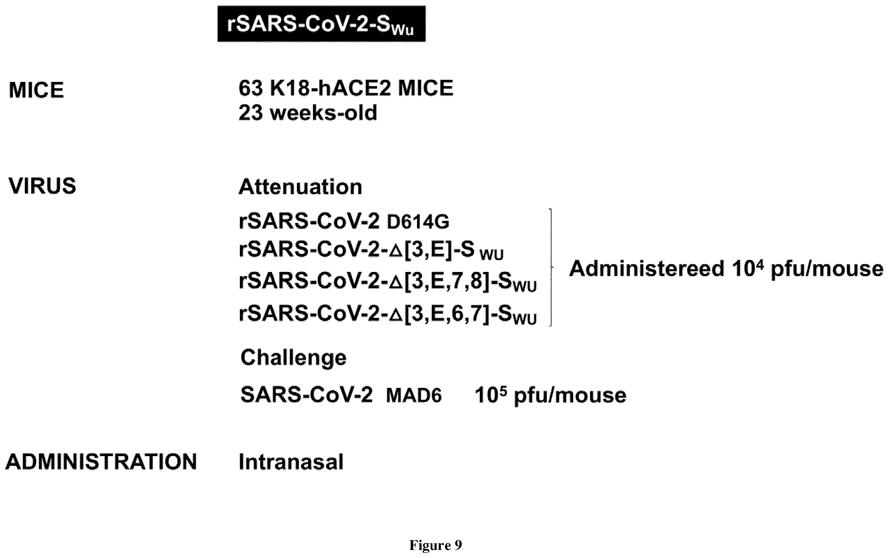

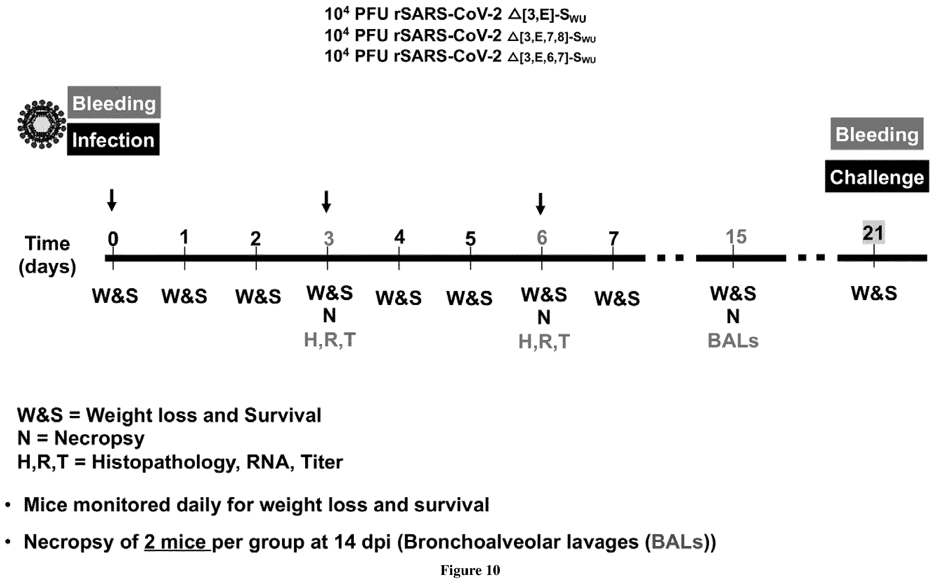

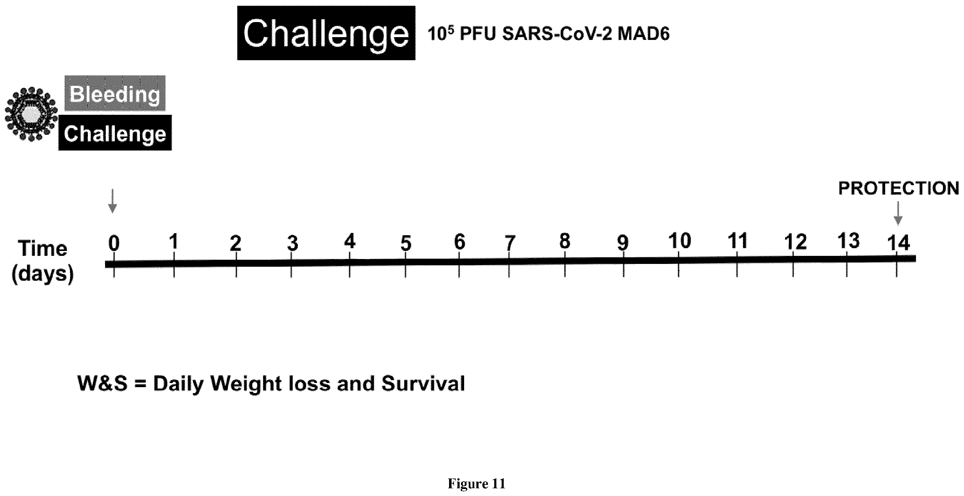

- **Figures 9-11:**: **Details of protection experiments performed in mice to determine the efficacy of the immunization by the RNA replicons developed.**

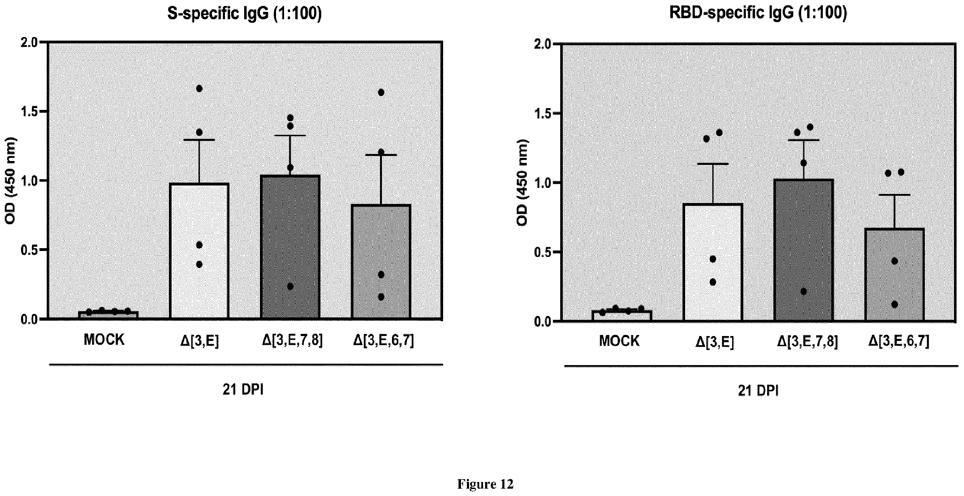

- **Figure 12:**: **SARS-CoV-2-Δ[3,E], SARS-CoV-2-Δ[3,E,7,8], SARS-CoV-2-Δ[3,E,6,7] RNA replicons induced generation of spike-specific and RBD-specific IgG antibodies in immunized mice.** Serum samples from immunized and non-immunized mice, were collected at 21 dpi and analyzed (n = 4 mice). Mice were immunized with the indicated RNA replicons encapsidated inside virus like particles (VLPs) derived from SARS-CoV-2. The absorbance (420 nm), provided by serum samples diluted 1:100, obtained by ELISA plate reader. The figure illustrates IgG levels. Results are represented as the mean ± SD.

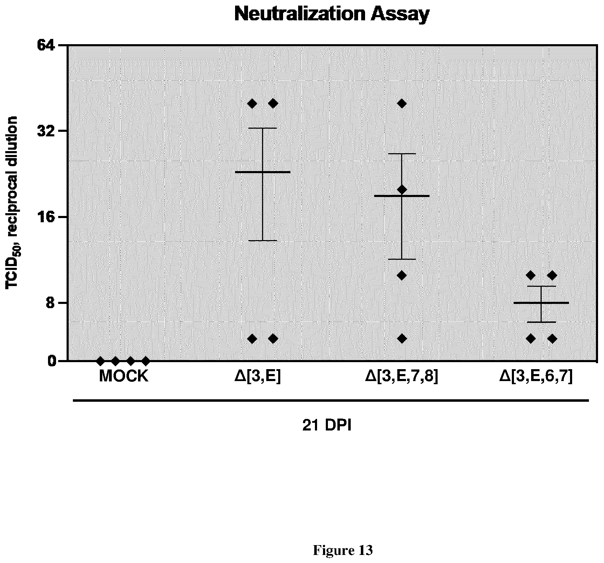

- **Figure 13:**: **SARS-CoV-2-Δ[3,E], SARS-CoV-2-Δ[3,E,7,8], SARS-CoV-2-Δ[3,E,6,7] RNA replicon induced neutralization of SARS-CoV-2 WU-WT.** Mice were immunized with the indicated RNA replicons encapsidated inside virus like particles (VLPs) derived from SARS-CoV-2. Serum samples from immunized and non-immunized mice, collected at 21 dpi were analyzed (n = 4 mice/assay). Serum samples diluted from 1:5 to 1:160 were incubated with 100 PFU of SARS-CoV-2 WU-WT. Results are represented as the mean ± SD. Results are illustrated in **Figure 13** and show that neutralization is more discriminatory than identification of specific antibodies as shown.

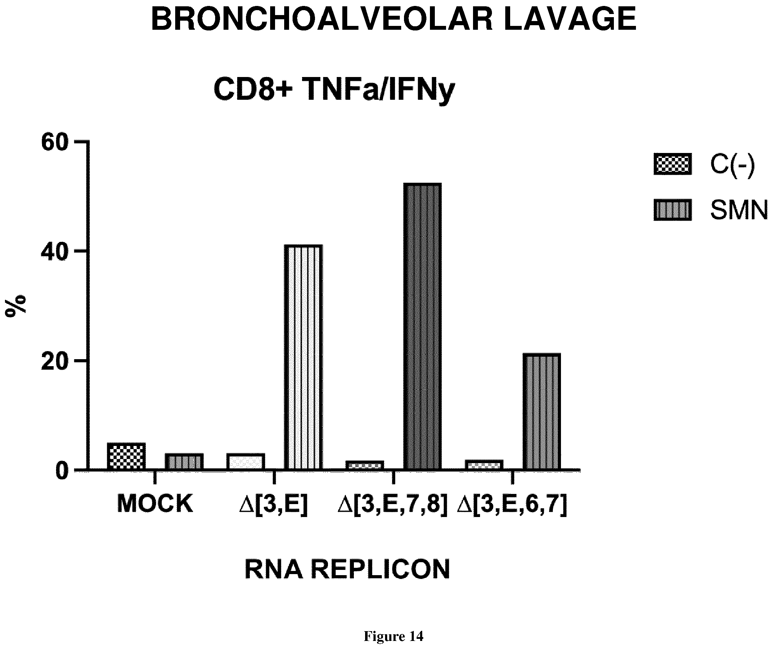

- **Figure 14:**: **Specific T-Cell response against a pool of SARS-CoV-2 antigens.** Mice were either immunized with SARS-CoV-2-Δ[3,E], SARS-CoV-2-Δ[3,E,7,8], or SARS-CoV-2-Δ[3,E,6,7] replicons encapsidated inside virus like particles (VLPs) derived from SARS-CoV-2, or mock-immunized. To analyze the immune response to the VLP replicons, mice were sacrificed at 21 days post-immunization, and the CD8+/TNFα+ and CD8+/IFNγ+ T-cell responses generated against a pool of peptides from the spike protein, were analyzed by flow cytometry. The SARS-CoV-2 S protein peptide pools used in the immunogenicity analysis (PM-WCPV-S-2. PepMix^{™} SARS-CoV-2 Spike Glycoprotein) were purchased from JPT Peptide Technologies (Berlin, Germany). JPT The S peptide pools were distributed in two groups spanning the S1 and S2 regions of the S protein, with each peptide pool containing 158 (S1) or 157 peptides (S2) as consecutive 15-mers overlapping by 11 amino acids. Bronchoalveolar lavage cells (BAL) were treated with RPMI 10% FBS (negative control) or a pool of antigens. This result showed a clearly activated and expanded population of cytotoxic T-Cells specific for SARS-CoV-2 antigens. C-, tissue culture media (RPMI); SMN, peptide pool from S1 and S2 proteins.

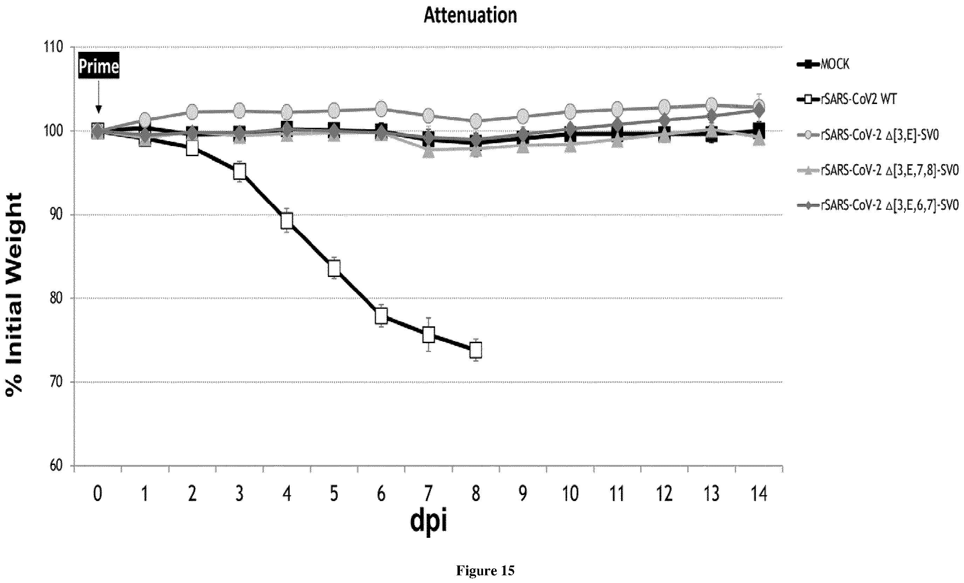

- **Figure 15-16:**: **Attenuation of SARS-CoV-2-Δ[3,E], SARS-CoV-2-Δ[3,E,7,8], SARS-CoV-2-Δ[3,E,6,7] in transgenic hACE2 mice.** Mice were mock inoculated (black squares) or inoculated intranasally with 10⁴ PFU of one of the three replicons (n = 4 mice). Weight loss and survival rate were daily monitored. The values indicate the mean value ± SEM. The results show that whereas WT virus induced important weight losses and that all mice died after infection, all those administered with the three RNA replicons administered as VLPs survived, indicating their safety.

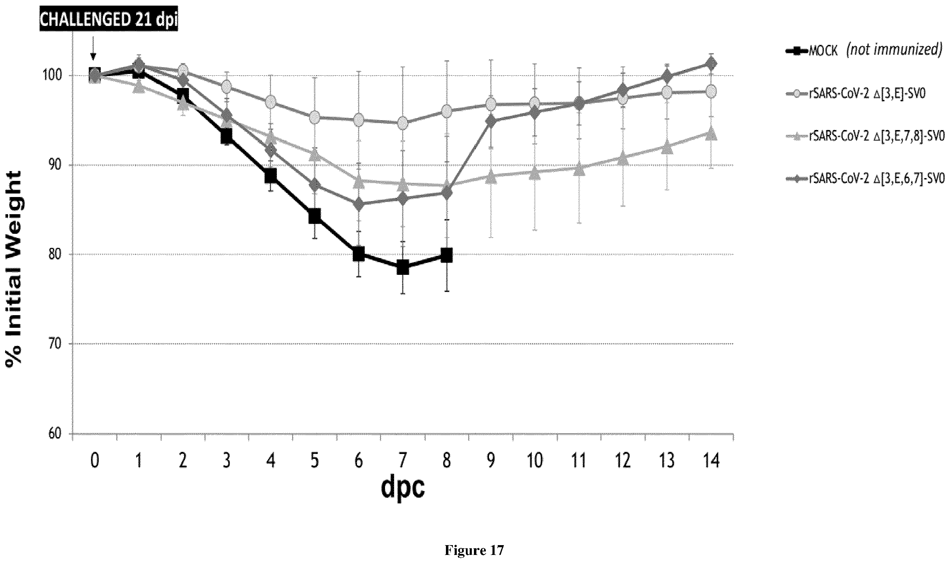

- **Figure 17-18:**: **Protection conferred by SARS-CoV-2-Δ[3,E], SARS-CoV-2-Δ[3,E,7,8], SARS-CoV-2-Δ[3,E,6,7] in transgenic hACE2 mice.** Mice were mock inoculated (black squares) or inoculated intranasally with 10⁴ PFU of one of the three replicons. After 21 days, mice were challenged intranasally with 10⁵ PFU/mice of the virulent SARS-CoV-2 virus. Weight loss and survival rate were daily monitored. The values indicate the mean value ± SEM. Results showed that 100% of the mice immunized only once with RNA replicons SARS-CoV-2-Δ[3,E] and SARS-CoV-2-Δ[3,E,7,8] survived, and 75% of mice administered SARS-CoV-2-Δ[3,E,6,7] also survived. In contrast, 100% of the non-immunized mice died.

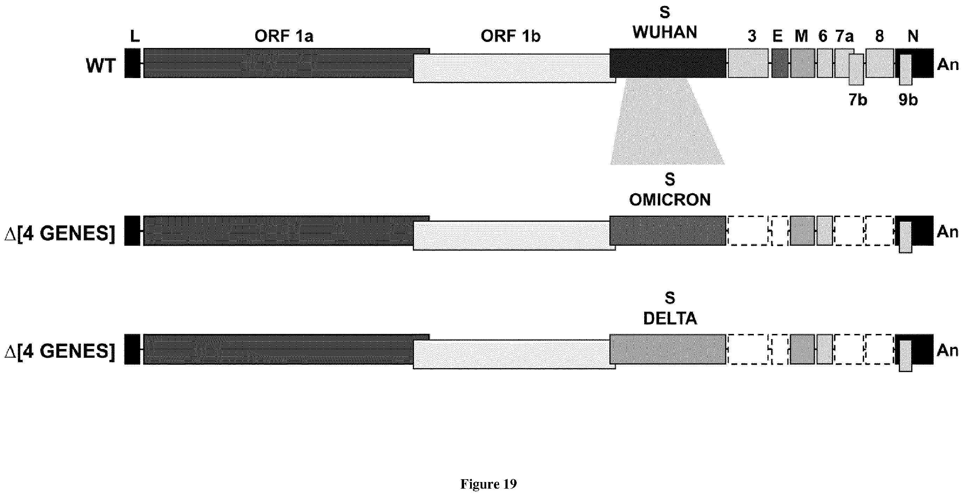

- **Figure 19:**: **Structural overview over SARS-CoV-2 replicon comprising sequences encoding an S protein from Omicron or Delta variant of SARS-CoV-2.** Schematic representation of the wild-type SARS-CoV-2 virus (upper panel) and polynucleotides containing 4 deletions as well as Omicron (middle panel) or Delta (bottom panel) S genes.

### EXAMPLES

### 1) ENGINEERING AN INFECTIOUS FULL-LENGTH cDNA OF SARS-CoV-2 VIRUS GENOME

The first requirement for the assembly of an RNA replicon derived from SARS-CoV-2 is the construction of a full-length infectious cDNA clone of the virus. The organization of viral genes in a coronavirus (CoV) genome is: 5' untranslated region (UTR) - replicase/transcriptase - spike protein (S) gene - envelope protein (E) gene - membrane protein (M) gene - nucleocapsid protein (N) gene - 3' UTR and polyA tail. The four structural proteins (S, E, M and N) contribute to the assembly of viral particles. In addition, coronavirus genome also contains genes encoding genus-specific accessory proteins. These proteins are involved in counteracting host defenses.

The cDNA encoding SARS-CoV-2 genome, Wuhan-Hu-1 strain (GenBank accession MN908947.3; sequence shown in SEQ ID NO: 1), was divided in six fragments (F1 to F6) that were chemically synthesized by GenScript (Piscataway, NJ, USA). These fragments covered the full-length viral genome SARSCoV2-FL (**Figure 1**) and (**Table 2**).

**Table 2. DNA fragments designed for SARS-CoV-2 cDNA assembly**

| | | | RESTRICTION SITES | | POSITION (nt) | |
|---|---|---|---|---|---|---|
| N° | SEQ ID NO: | SIZE (bp) | 5' END | 3' END | GENOME ^{(a)} | pBAC-SARS-CoV-2-FL ^{(b)} |
| F1 | SEQ ID NO: 9 | 957 | Ascl | BsiWl | 1 - 346 | 7279 - 8235 |
| F2 | SEQ ID NO: 10 | 6401 | BsiWl | Pmel | 347 - 6747 | 8236 - 14636 |
| F3 | SEQ ID NO: 11 | 7209 | Pmel | Mlul | 6748 - 13956 | 14637 - 21845 |
| F4 | SEQ ID NO: 12 | 6130 | Mlul | SanDl | 13957 - 20086 | 21846 - 27975 |
| F5 | SEQ ID NO: 13 | 5227 | SanDl | BamHl | 20087 - 25313 | 27976 - 33202 |
| F6 | SEQ ID NO: 14 | 4612 | BamHl | Rsrll | 25314 - 29870 | 33203 - 37814 |

| | | | | | | |
|---|---|---|---|---|---|---|
| (a) Nucleotide numbering in agreement with GenBank sequence MN908947, where 1 is the first nucleotide. (b) Nucleotide numbering in agreement with pBAC infectious cDNA sequence, were virus starts in nt 7890 from pBAC-SARS-CoV-2-FL (SEQ_ID 8) | | | | | | |

pBeloBAC11 plasmid (pBAC) is a commercially available vector and was used to clone the cDNA of SARS-CoV-2 (SEQ ID NO: 1). This plasmid (7507 bp) contains the origin of replication of *E.coli,* factor F (oirS), the chloramphenicol resistance gene (cat) and genes required to maintain a single copy of the plasmid per cell (parA, parB, parC and repE). This vector allows the stable maintenance of large DNA fragments in bacteria. The pBAC plasmid including the full-length cDNA of SARS-CoV-2 was named pBAC-SARS-CoV-2-FL (SEQ ID NO: 8).

Two silent mutations were introduced as genetic markers: A20085>G, generating a unique SanDI restriction site, and G26840>C, eliminating Mlul and BsiWI restriction sites (**Figure 1**). These genetic markers are very useful as, according to NextStrain analysis, only a few cases (one in Egypt and two in Mexico) with the A20085>G mutation, which did not carry the other genetic marker, have been identified. And no cases with the G26840>C mutation have been identified in the more than five million SARS-CoV-2 sequences available to date.

In addition, viral genome cDNA was flanked by cytomegalovirus promoter (at the 5' end) and the hepatitis delta virus ribozyme sequence together with the bovine growth hormone polyadenilation and termination signals (at the 3' end) (**Figure 1**).

The DNA fragments were sequentially cloned into a bacterial artificial chromosome (BAC) (Almazan et al., 2000) that was used as a vector for SARS-CoV-2 genome maintenance and amplification, similarly as previously described by (Almazan et al., 2014). This BAC was amplified in *Escherichia coli* DH10B bacteria, non-pathogenic. All cloning steps were verified by restriction pattern and sequencing with primers designed across several regions of SEQ ID NO: 1. An expert in the field would know how to design the appropriate primers spanning the whole nucleotide sequence.

To rescue the recombinant infectious virus, the BAC that included the full-length cDNA of the virus plus regulatory sequences was purified using the large construct kit (Qiagen), following the manufacturer's instructions. Briefly, infectious cDNA was transfected into baby hamster kidney (BHK21) cells using Lipofectamine 2000 (ThermoFisher Scientific), following the manufacturer's recommendations. Six hours after transfection BHK21 cells were detached from the plate and were seeded over confluent Vero E6 or VeroE6-TMPRSS2 cells, susceptible to SARS-CoV-2 infection. At 48 to 72 hours post-transfection, culture supernatant, containing the recombinant rSARS-CoV-2 virus, was collected and stored as passage 0.

### 2) ENGINEERING RECOMBINANT SARS-COV-2 MUTANTS WITH DELETIONS IN ONE OR MORE GENES

DNA fragments were designed (**Table 3**) and chemically synthesized by GenScript (Piscataway, NJ, USA). These fragments contained the deletion of one or several viral genes. These deletions were combined to obtain different mutant viruses or replicons described below.

**Table 3. DNA fragments required for the engineering of SARS-CoV-2 deletion mutants.**

| | RESTRICTION SITES | | | |
|---|---|---|---|---|
| NAME | SIZE (bp) ^{(a)} | 5' END | 3' END | DELETION (nt) ^{(b)} |
| Fdel3 | 256 | BamHl | Hpal | 25385 - 26206 |
| FdelE | 386 | BspEI | Agel | 26237 - 26452 |
| Fdel3-E | 376 | BamHl | Agel | 25385 - 26452 |
| Fdel6 | 438 | PpuMI | BmgBl | 27202 - 27367 |
| Fdel7 | 1229 | PpuMI | Avrll | 27388 - 27759 |
| Fdel7a | 1310 | BmgBI | Avrll | 27388 - 27759 |
| Fdel7b | 1493 | BmgBl | Avril | 27768 - 27876 |
| Fdel8 | 644 | BmgBl | Avril | 27888 - 28240 |
| Fdel67 | 954 | PpuM! | Avril | 27192 - 27845 |
| Fdel78 | 756 | PpuMI | Avrll | 27388 - 28238 |
| Fdel6-7-8 | 563 | PpuMI | Avril | 27202 - 28239 |

| | | | | |
|---|---|---|---|---|
| (a) Refers to the sub-fragment within the pSL-F6-Bam-Avr intermediate plasmid, because it contains the corresponding deletions, is smaller than the full F6 sequence; (b) In agreement with SEQ ID NO: 1. | | | | |

To engineer these mutants, pBAC-F6 plasmid (**Table 2**) SEQ ID NO: 14 was digested with BamHI and Avrll. The resulting 3296 bp fragment, containing nucleotides 25314 to 28609 from SARS-CoV-2 genome (SEQ ID NO: 1), was cloned in the same restriction sites of commercial plasmid pSL1190 (Amersham) and intermediate plasmid pSL-F6-Bam-Avr was obtained. Subsequently, each of the mutant fragments (**Table 3**) was cloned in the indicated restriction sites, which were unique in pSL-F6-Bam-Avr plasmid, leading to intermediate plasmids pSL-F6-del3, pSL-F6-delE, pSL-F6-del[3,E], pSL-F6-del6, pSL-F6-del7, pSL-F6-del7a, pSL-F6-del7b, pSL-F6-del8, pSL-F6-del[6,7] and pSL-F6-del[7,8] and pSL-F6-del[6,7,8]. Fdel8 fragment was introduced in the BmgBl and Avrll pSL-F6-del6 restriction sites, leading to plasmid pSL-F6-del[6,8]. Afterwards, Fdel3-E fragment (**Table 3**) was introduced in BamHI and Agel restriction sites from pSL-F6-del6, pSL-F6-del7, pSL-F6-del8, pSL-F6-del[6,8], pSL-F6-del67 and pSL-F6-del78, leading to plasmids pSL-F6-del[3,E,6], pSL-F6-del[3,E,7], pSL-F6-del[3,E,8], pSL-F6-del[3,E,6,8], pSL-F6-del[3,E,6,7] and pSL-F6-del[3,E,7,8], respectively.

Each of the intermediate pSL-F6 plasmids were digested with BamHI and Avrll and the inserts were cloned in the same restriction sites from plasmid pBAC-F6, leading to plasmids pBAC-F6-Δ3, pBAC-F6-ΔE, pBAC-F6-Δ[3,E], pBAC-F6-Δ[3,E,6], pBAC-F6-Δ[3,E,7], pBAC-F6-Δ7a, pBAC-F6-Δ7b, pBAC-F6-Δ[3,E,8], pBAC-F6-Δ[3,E,6,8], pBAC-F6-Δ[3,E,6,7] and pBAC-F6-Δ[3,E,7,8] and pBAC-F6-Δ[3,E,6,7,8]. Finally, these plasmids were digested with BamHI and Rsrll and the inserts were introduced into the same restriction sites from SARS-CoV-2 infectious cDNA, leading to infectious clones pBAC-SARSCoV2-Δ3, pBAC-SARSCoV2-ΔE, pBAC-SARSCoV2-Δ[3,E], pBAC-SARSCoV2-Δ[3,E], pBAC- SARSCoV2-Δ[3,E,6], pBAC- SARSCoV2-Δ[3,E,7], pBAC-SARSCoV2-Δ[3,E,8], pBAC- SARSCoV2-Δ[3,E,6,8], pBAC- SARSCoV2-Δ[3,E,6,7] and pBAC- SARSCoV2-Δ[3,E,7,8].

The exact deletions of some of these mutants are in reference to SEQ ID NO: 1:
- SARS-CoV-2-Δ[3] from 25385nt to 26206nt;
- SARS-CoV-2-Δ[E] from 26237nt to 26452nt;
- SARS-CoV-2-Δ3E from 25385nt to 26452nt;
- SARSCoV2-Δ[3,E,6,7] from 25385nt to 26452nt, and from 27192nt to 27845nt); and
- SARSCoV2-Δ[3,E,7,8] from 25385nt to 26452nt, and from 27388nt to 28238nt (**Figures 2** **and** **4**).

The nucleotide sequence of SARS-CoV-2-Δ[3] is SEQ ID NO: 15, of SARS-CoV-2-Δ[E] is SEQ ID NO: 16, of SARS-CoV-2-Δ[3,E] is SEQ ID NO: 17, of SARS-CoV-2-Δ[3,E,6] is SEQ ID NO: 47, of SARS-CoV-2-Δ[3,E,7] is SEQ ID NO: 48, of SARS-CoV-2-Δ7a is SEQ ID NO: 20, of SARS-CoV-2-Δ7b is SEQ ID NO: 21, of SARS-CoV-2-Δ[3,E,8] is SEQ ID NO: 49, of SARS-CoV2-Δ[3,E,6,8] is SEQ ID NO: 44, of SARS-CoV2-Δ[3,E,6,7] is SEQ ID NO: 42 and of SARS-CoV2-Δ[3,E,7,8] is SEQ ID NO: 43.

### 2.1 Engineering a SARS-CoV-2 derived replicon with additional small deletions in ORF1a

ORF nsp1 maps at the 5'-end of the coronavirus genome, whereas alternative attenuating deletion or those contributing to the replication-competent propagation deficient development of RNA replicons derived from SARS-CoV-2 map at distal positions of ORF1, which makes it very unlikely that a single recombination event could restore virulence or propagation ability, therefore increasing the safety of the RNA polynucleotides disclosed herein. Different individual or combined domains of ORF nsp1 were deleted, named A, B, C, or D, mapping at positions in the virus genome (SEQ ID NO: 1): 284nt to 307nt (A), 500 nt to 532nt (B), 629nt to 655nt (C), 728nt to 760nt (D).

We generated the deletions A to D individually or a combination of two of them, and the optimum combination to attenuate the SARS-CoV-2 were the deletion of domain nsp1-A (nsp1-Δ[A]), or the double deletion of domains B and D (nsp1-Δ[B,D]). To engineer the deletions termed nsp1-Δ[A] and nsp1-Δ[B,D], two synthetic fragments containing the nsp1 deletions were designed and chemically synthesized by Thermo Fisher Scientific, fragment Fnsp1-ΔA (SEQ ID NO: 45) containing nucleotides 1 to 351 of the SARS-CoV-2 genome (SEQ ID NO: 1) with the deletion of nsp1 domain A, and fragment Fnsp1-Δ[B,D] (SEQ ID NO: 46) containing nucleotides 346 to 6751 of SARS-CoV-2 genome (SEQ ID NO: 1) with the combined deletion of nsp1 domains B and D.

To engineer the replicons containing selected mutations in nsp1, the Fnsp1-ΔA fragment was digested with Ascl and BsiWI and the resulting 933bp fragment was cloned into the same sites of pBAC-SARS-CoV-2-Δ[3, E,6,7] ( SEQ ID NO: 42) and pBAC-SARS-CoV-2-Δ[3,E,7,8] ( SEQ ID NO: 43) replicon candidates. The Fnsp1-Δ[B, D] fragment was digested with BsiWI and Pmel and the resulting 6335 bp fragment was cloned into the same sites of pBAC-SARS-CoV-2-Δ[3,E,6,7] ( SEQ ID NO: 42) and pBAC-SARS-CoV-2-Δ[3,E,7,8] (SEQ ID NO: 43) replicon candidates.

### 2.2 Transfection of cDNAs and recovery of infectious virus of SARS-CoV-2 deletion mutants and replicons.

To rescue wild type viruses and deletion mutants, Vero E6-TMPRSS2 cells grown at 95% confluence in 12.5 cm² flasks were transfected with 6 µg of each cDNA clone using Lipofectamine 2000 (Invitrogen, Thermo Fisher Scientific) in the proportion 1:3 (micrograms : microliters) of ADN:*Lipofectamine 2000*, according to the manufacturer's specifications. Six hours later, medium containing Lipofectamine complexes was removed and replaced by fresh medium. After 72 h incubation at 37°C, cell supernatants were harvested (passage 0), passaged once on fresh Vero E6-TMPRSS2 cells (passage 1) and then, the titers of virus stocks were determined by plaque assay on Vero E6 cells. The complete genome sequence of viral stocks was determined to confirm that recombinant viruses had been rescued correctly. Viral stocks were stored at -80 °C.

### 2.3 Packaging Cell Line for Amplification of SARS-CoV-2 Deletion Mutants and replicons in vitro

In order to grow RNA replicons SARS-CoV-2-Δ[3,E], SARS-CoV-2-Δ[3,E,6,7], SARS-CoV-2-Δ[3,E,6,8] and SARS-CoV-2-Δ[3,E,7,8], proteins 3a and E were provided in trans by using the expression plasmid shown in **Figure 6** of PCT/EP2022/083289 or by stably transformed cells expressing these proteins.

In order to asses if the single deletion of gene E or gene 3a would impair virus propagation, the growth kinetics of SARS-CoV-2-Δ[3] and SARS-CoV-2-Δ[E] was analyzed in Vero E6-TMPRSS2 cells at 24, 48 and 72 hpi. These mutants grew somehow slower and gave rise to lower titers (**Figure 7** of PCT/EP2022/083289).

Two different cell lines expressing E and ORF3a genes were generated to rescue and amplify SARS-CoV-2 replicons lacking E and ORF3a genes: VeroE6-[E-IRES-ORF3a] and VeroE6-TMPRSS2-[E-IRES-ORF3a]. E and ORF3a genes were cloned into a pLVX-TetOne-Puro plasmid (Takara) under the control of a tetracycline-inducible promoter (**Figure 6** of PCT/EP2022/083289). The lentiviral vector LVX-TetOne-Puro-[E-IRES-ORF3a] was used following manufacturer instructions. VeroE6 and VeroE6-TMPRSS2 cells were transduced with LVX-TetOne-Puro-[E-IRES-ORF3a] lentiviral vector. Selection of transduced cells started 48 hours post-transduction by adding puromycin to the media. Two weeks later puromycin-resistant individual clones were isolated and amplified. Expression of E and ORF3a proteins was tested by western blot in the presence or the absence of the inductor (doxycycline) to validate the selected clones.

VeroE6 cells were provided by E. Snijder (University of Leiden, the Netherlands). VeroE6-TMPRSS2 cells were obtained from the Centre For AIDS Reagents (National Institute for Biological Standards and Control, United Kingdom). Cells were grown in Dulbecco's modified Eagle's medium (DMEM) with 25 mM 4-(2-hydroxyethyl)-1-piperazineethanesulfonic acid and 4.5 g/L glucose (BioWhittaker; Lonza), supplemented with 4 mM glutamine, 1× nonessential amino acids (Sigma-Aldrich), and 10% vol/vol fetal bovine serum (FBS; HyClone; Thermo Scientific).

VeroE6 or VeroE6-TMPRSS2 cells were grown to 95% confluence in 12.5-cm² flasks and transfected with 6 µg of each infectious cDNA clone and 18 µL of Lipofectamine 2000 (Invitrogen), according to the manufacturer's specifications. Three independent cDNA clones were recovered of each mutant. At 6 h post-transfection (hpt), cells were washed with PBS 1X, and incubated at 37 °C for 72 h (passage 0) with fresh media. Cell supernatants were harvested and passaged two times on fresh cells (passages 1 and 2). The viability, titer, and sequence of the mutants were analyzed to generate viral stocks for in vitro and in vivo evaluations.

To rescue viruses lacking the E and ORF3a genes, VeroE6-[E-IRES-ORF3a] or VeroE6-TMPRSS2-[E-IRES-ORF3a] were transfected with SARS-CoV-2 replicon cDNAs. At 6 hpt, the medium containing the plasmid-Lipofectamine complexes was removed from the transfected cells and washed. Fresh medium supplemented with doxycycline at a concentration of 1 µg/mL was added and cells were incubated at 37 °C for 72 h. For successive virus amplification passages and virus stocks, doxycycline at a concentration of 1 µg/mL was added to VeroE6-[E-IRES-ORF3a] or VeroE6-TMPRSS2-[E-IRES-ORF3a] to induce expression of E and ORF3a genes to allow propagation of SARS-CoV-2 replicons. Results are illustrated in Figure 3 and show that the cells express the proteins needed for complementation of the polynucleotides encoding the propagation-deficient SARS-CoV-2 deletion mutants described above.

To study growth kinetics subconfluent monolayers (90% confluence) of VeroE6, VeroE6-TMPRSS2, Huh-7 and Huh-7-ACE2 cells, or Calu-3a cells, grown in 12-well plates were infected at an MOI of 0.001 with the indicated viruses. Culture supernatants were collected at 0, 24, 48, and 72 hpi, and virus titers were determined by plaque assay.

Among the two cell lines, only VeroE6-TMPRSS2-[E-IRES-ORF3a] was selected for further analysis, since growth kinetics and titers of SARS-CoV-2-WT in VeroE6-TMPRSS2 were faster and higher, respectively. Therefore, VeroE6-TMPRSS2-[E-IRES-ORF3a] were transfected with different combination of genes deleted SARS-CoV-2-Δ[3,E], SARS-CoV-2-Δ[3,E,6,7], or SARS-CoV-2-Δ[3,E,7,8] replicons. At 72 hpt (passage 0) supernatants were harvested and passed once more (passage 1) to evaluate rescue, growth and amplification of this vaccine candidate.

In the absence of doxycycline (-E/-ORF3a) no SARS-CoV-2-Δ[3,E], SARS-CoV-2-Δ[3,E,6,7], or SARS-CoV-2-Δ[3,E,7,8] could be detected by focus-forming immunofluorescence assay, while in its presence titers of 10⁴ and 10⁵ FFU/mL (focus-forming unit) were detected at passages 0 and 1, respectively. This result validates the system for the rescue, growth and amplification of SARS-CoV-2 replicons for its in vivo evaluation.

The cells transformed with these plasmids can thus be used to complement the deletion mutants.

### 2.4 Titration of SARS-CoV-2 Replicons by Focus-Forming Immunofluorescence Assay.

In total, 5 × 10⁴ VeroE6-TMPRSS2 cells were seeded per well in 96-well plates in 100 µL of media 1 d prior to the immunofluorescence assay. The next day, cells were infected with 20 µL of undiluted or serial 10-fold-diluted virus. At 16 hpi, cells were fixed with paraformaldehyde 4% wt/vol for 40 min, washed, and permeabilized with chilled methanol at R/T for 20 min. Nonspecific binding was blocked with FBS 10% in PBS for 1 h at R/T. Then, cells were incubated for 90 min at R/T with rabbit monoclonal antibody anti-N-SARS-CoV/SARS-CoV-2 (SinoBiological). Secondary monoclonal antibody goat anti-rabbit conjugated with Alexa 488 (Invitrogen) was incubated for 45 min to detect and count infectious foci of SARS-CoV-2 replicons. The titer was expressed as focus-forming units (FFUs) per milliliter.

VeroE6-TMPRSS2-[E-IRES-ORF3a] were transfected with the SARS-CoV-2, SARS-CoV-2-Δ[3,E], SARS-CoV-2-Δ[3,E,7,8], SARS-CoV-2-Δ[3,E,6,8] or SARS-CoV-2-Δ[3,E,6,7] replicon. At 72 hpt (passage 0) supernatants were harvested and passed four times (passage 1 to 4) evaluating rescue, growth and amplification of this vaccine candidate. Titers were detected at passages 0, 1, 2 and 4. The replicons characterized by four deleted genes provided the highest titers of which SARS-CoV-2-Δ[3,E,7,8] and SARS-CoV-2-Δ[3,E,6,7] were selected for further analysis (**Figure 5**). The results demonstrate the reproducibility and stability of the different RNA replicons during sequential passage.

### 2.5 Viral Titration by Plaque Formation Assay

Vero E6 cells were seeded on 12-well plates, grown to 100% confluence and infected by duplicate with factor 10 serial dilutions of viral supernatants. After 45 min adsorption at 37°C, the inoculum was removed and cells were overlaid with DMEM supplemented with 4 mM glutamine, 1% v/v of non-essential amino-acids, 2% v/v of FBS, 0.16 mg/ml of DEAE-Dextran and 1% low-melting agarose. 96 hpi, cells were fixed with 10% formaldehyde and stained with 0.1% crystal violet. The number of plaques formed in each well was determined. Titers were determined by multiplying the number of plaques in each well by the dilution factor and expressed as the number of plaque forming units (PFUs) per ml (PFU/ml).

In order to examine the stability of the SARS-CoV-2-Δ[3,E], SARS-CoV-2-Δ[3,E,6,7], or SARS-CoV-2-Δ[3,E,7,8] replicons in cell culture and also assess whether it could recombine with the RNA encoding E and ORF3a proteins transcribed in the packaging cell lines, RNA from cell culture was extracted, and the region between the S and N genes within the SARS-CoV-2-Δ[3,E,7,8] or alternative replicons was amplified by PCR and sequenced with primers WH-25155-VS (SEQ ID NO: 26) and WH-28957-RS (SEQ ID NO: 27). After 16 passages in VeroE6-[E-IRES-ORF3a] or VeroE6-TMPRSS2-[E-IRES-ORF3a], we found that it remained genetically stable with no evidence that SARS-CoV-2 replicon deletion mutants recombined with the RNA encoding the E or ORF3a proteins.

The stability of rSARS-CoV-2-nsp1-Δ[A] and rSARS-CoV-2-nsp1-Δ[B,D] mutants was analyzed in VeroE6-TMPRSS2 cells. Cells were seeded in 12.5-cm² flasks and infected with the mutant. Every 24 h, a third of the supernatant was used to infect a fresh cell monolayer. After each passage, the remaining supernatant was stored at -80°C. Cells were lysed to extract RNA as described above. Full-length virus was sequenced to show the presence of the introduced deletion. The results indicated that after 5 passages of the virus including the deletions of part of nsp1 gene (ΔA or Δ[B,D]) the virus was competent in replication and maintained its sequence.

### 2.6 Updated Spike Protein in Replicon

It is well known in the art that SARS-CoV-2 Omicron strain is prevalent in the world today. In fact, in the USA more than 90% on novel infections are caused by the Omicron strain. In addition, the observation has been made that immunization with only Omicron strains induced good protection against Omicron strains, but not against older strains. On the other hand, exclusive immunization with older strains, such as Delta, induced good protection against the infection by all earlier strains, but reduced protection against the highly evolved Omicron strain.

For this reason, a vaccine strain was produced that includes S proteins from Omicron and Delta strains. SARS-CoV-2 deletion mutant cDNAs were used to introduce the new Spike sequences (Delta and Omicron variants). First, two F4 DNA fragments were obtained by chemical synthesis (GeneScript) (F4-S Delta, SEQ ID NO: 40; and F4-S Omicron, SEQ ID NO: 41) that included nucleotides from 20084 to 25312 of SARS-CoV-2 genome, flanked by SanDI and BamH! unique restriction sites. The fragments digested with SanDI and BamHl were introduced into the corresponding sites of plasmid pBAC^{FL}-SARS-CoV-2-Δ[3,E,6,7] or pBAC^{FL}-SARS-CoV-2-Δ[3,E,7,8], to generate the corresponding cDNA infectious clones (pBAC^{FL}-SARS-CoV-2-Δ[3,E,6,7]-S_{omicron}, pBAC^{FL}-SARS-CoV-2-Δ[3,E,6,7]-S_{delta}, pBAC^{FL}-SARS-CoV-2-Δ[3,E,7,8]-S_{omicron} and pBAC^{FL}-SARS-CoV-2-Δ[3,E,7,8]-S_{delta}). This method can also be used for producing strains comprising S proteins from Omicron and Delta strains for other SARS-CoV-2 deletion mutants described herein. The integrity of the cloned DNA was verified by restriction pattern analysis and by Sanger sequencing.

### 3) RESCUE OF RNA REPLICONS BY REVERSE TRANSFECTION

Rescue of the RNA replicon SARS-CoV-2 Δ[3,E,6,7] was determined when applying the direct and reverse transfection method and complementation of the 3a and E protein in trans. Application of the reverse transfection method leads to increased RNA replicon titers compared to the direct transfection method.

In total, 5 × 104 VeroE6-TMPRSS2 cells were seeded per well in 96-well plates in 100 µL of media 1 d prior to the immunofluorescence assay. The next day, cells were infected with 20 µL of undiluted or serial 10-fold-diluted virus. At 16 hpi, cells were fixed with paraformaldehyde 4% wt/vol for 40 min, washed, and permeabilized with chilled methanol at R/T for 20 min. Nonspecific binding was blocked with FBS 10% in PBS for 1 h at R/T. Then, cells were incubated for 90 min at R/T with rabbit monoclonal antibody anti-N-SARS-CoV/SARS-CoV-2 (SinoBiological). Secondary monoclonal antibody goat anti-rabbit conjugated with Alexa 488 (Invitrogen) was incubated for 45 min to detect and count infectious foci of SARS-CoV-2 replicons. The titer was expressed as focus-forming units (FFUs) per milliliter.

### 4) GENERATION OF VIRUS TITER STOCK OF CONCENTRATED SARS-COV-2 VLPS

For generation of viral stocks SARS-CoV-2-Δ[3,E], SARS-CoV-2-Δ[3,E,7,8], SARS-CoV-2-Δ[3,E,6,7] RNA replicons VeroE6-TMPRSS2 were infected at MOI 0.001 and the 3a and E proteins provided in trans. The supernatants were recovered at 3 dpi and further processed using the Intact Virus Precipitation Reagent (Invitrogen) according to manufacturer's instructions to obtain a non-concentrated (NC) and concentrated (C) VLP stock. VLP stocks were titrated by immunofluorescence technique as described above. Results are represented as the mean ± SD (**Figure 8**).

### 5) EVALUATION OF GROWTH AND VIRULENCE OF SARS-CoV-2 DELETION MUTANTS IN VIVO.

The attenuation of the mutants was evaluated in K18-hACE2 C57BL/6J mice (strain 2B6.Cg-Tg(K18-ACE2)2Prlmn/J, 20-26-week-old) obtained from The Jackson Laboratory (Bar Harbor, ME, USA). Mice were mock inoculated or inoculated intranasally with 10⁴ PFU of the SARS-CoV-2 Δ[3,E], SARS-CoV-2 Δ[3,E,7,8] or SARS-CoV-2 Δ[3,E,6,7] replicons. Five mice were used for monitoring the disease and six for lung sample collection at days 3 and 6 post-infection (n=3 mice at each time point).

Body weight and survival of infected mice were monitored for 10 days. Animals suffering weight losses as much as 20% of the initial weight were sacrificed according to the established end point criteria. At days 3 and 6 post-infection, three mice from each experimental group were sacrificed by cervical dislocation for lung sampling. Half of the right lung was collected for viral titer determination, and stored at -80 °C until use. The rest of the lung was stored in RNAlater solution (Sigma-Aldrich) for 48 h at 4°C for RNA extraction and stored at -80°C until further processing to guarantee the integrity of the RNA molecules. The left lung was fixed in a 10% zinc formalin solution (Sigma-Aldrich) for 24-48 hours at 4°C for virus inactivation and subsequent histopathological analysis.

To determine viral titers, lungs were homogenized in 2 mL of PBS containing 100 IU/mL penicillin, 0.1 mg/mL streptomycin, 50 µg/mL gentamicin, and 0.5 µg/mL amphotericin B (Fungizone) using a gentleMACS dissociator (Miltenyi Biotec, Inc.). Virus titrations were performed in VeroE6-TMPRSS2 cells as described above. Viral titers were expressed as PFU counts per gram of tissue. All work with infected animals was performed in an Animal Biosafety Level 3+ laboratory wearing personal protection equipment (3M).

### 5.1 Lifespan and Weight

The pathogenicity of **SARS-CoV-2-Δ[3,E], -Δ[3,E,6,7], -Δ[3,E,7,8]** replicons was evaluated in K18-hACE2 mice. SARS-CoV-2 was used as the reference virulent virus (WT); 1 × 10⁴ PFU of virus or replicon were intranasally inoculated into mice, and weight loss and survival were monitored for 14 days.

At days 3 and 6 post-infection, three mice from each experimental group were sacrificed by cervical dislocation for lung sampling. Half of the right lung was collected for viral titer determination, and stored at -80 °C until use. The rest of the lung was stored in RNAlater solution (Sigma-Aldrich) for 48 h at 4°C for RNA extraction and stored at -80°C until further processing to guarantee the integrity of the RNA molecules. The left lung was fixed in a 10% zinc formalin solution (Sigma-Aldrich) for 24-48 hours at 4°C for virus inactivation and subsequent histopathological analysis.

The results showed severe weight losses for mice infected with SARS-CoV-2 WT. However, mice infected with the SARS-CoV-2 Δ[3,E], SARS-CoV-2 Δ[3,E,7,8], SARS-CoV-2 Δ[3,E,6,7] replicons maintained weight over the 14 days post-infection. Survival rate of mice was additionally monitored showing death of all mice after 8 days post-infection when infected with SARS-CoV-2 WT. By contrast, mice infected with the RNA replicons lead to a survival rate of 100 % over the 14 days post-infection.

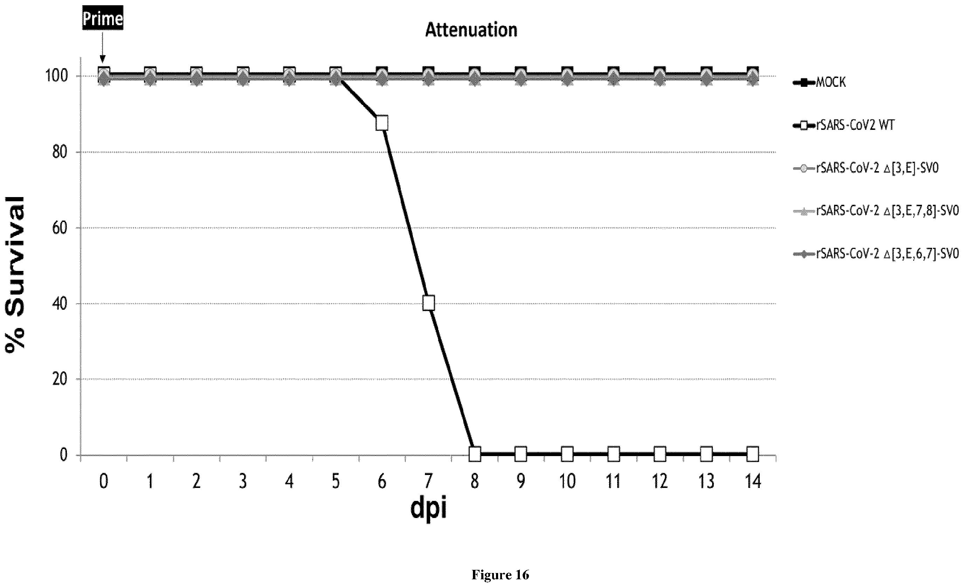

Full attenuation and thus safety of the SARS-CoV-2 Δ[3,E], SARS-CoV-2 Δ[3,E,7,8], SARS-CoV-2 Δ[3,E,6,7] replicons was demonstrated. The results showed that the replicons of the invention cause no pathology to the mice model used in the experiments and thus demonstrate safety when administered (**Figure 15****,** **Figure 16**). The safety was further demonstrated by the absence of propagation polynucleotides in immunized mice lungs (**Figure 6**).

### 5.2 Replication, Transcription and Propagation

To further characterize infected mice, virus titer or RNA levels were analyzed in lungs at 3 and 6 dpi. High virus titers were determined at 3 and 6 dpi in the lungs of mice infected with SARS-CoV-2-WT virus or the indicated deletion mutants.

To analyze safety of the replicons, 21-week-old K18-hACE-2 transgenic mice were either infected with SARS-CoV-2 WU-WT or with SARS-CoV-2 Δ[3,E], SARS-CoV-2 Δ[3,E,7,8] or SARS-CoV-2 Δ[3,E,6,7]. High virus titers were detected at 3 and 6 dpi in the lungs of mice infected with SARS-CoV-2 WU-WT virus, but no virus growth was observed in the lungs of mice inoculated with the SARS-CoV-2-Δ[3,E], SARS-CoV-2-Δ[3,E,7,8], SARS-CoV-2-Δ[3,E,6,7] replicons. In the absence of external complementation of the protein 3 and protein E, the constructed replicons did not spread from cell to cell. While the SARS-CoV-2 Δ[3,E], SARS-CoV-2 Δ[3,E,7,8] and SARS-CoV-2 Δ[3,E,6,7] were stably maintained in the cell (**Figure 5**), they cannot be propagated from one cell to another cell (**Figure 6**). The replicons of the present invention are thus considered to be propagation-defective, replication-competent RNA replicons.

The results therefore show that SARS-CoV-2 deletion mutants replicated in the sense of producing further RNA polynucleotides without spreading to other cells.

### 5.3. Extraction and Purification of RNA from Lung Samples

Lung samples were removed from the RNA storage solution and homogenized in 2 ml of RLT lysis buffer (Qiagen, Germany) with 1% v/v β-mercaptoethanol (Sigma-Aldrich) using a gentleMACS Dissociator homogenizer (Miltenyi Biotec), according to the manufacturer's instructions. The homogenized samples were centrifuged at 3000 rpm for 10 minutes at 4° C. Then, total RNA was purified from the supernatant using the RNeasy Mini Kit reagent (Qiagen).

### 5.4 Synthesis of cDNA from RNA by Reverse Transcription (RT)

cDNAs were synthetized by reverse transcription using the High-Capacity cDNA transcription kit (Applied Biosystems, USA), following the manufacturer's recommendation. 100 - 150 ng of total lung RNA were used as template and random hexamers as primers in a reaction volume of 20µl. cDNA products were subsequently subjected to PCR for sequencing using Vent polymerase (New England Biolabs). cDNA products from mouse lungs were analyzed by real-time quantitative PCR (qPCR) for viral RNA synthesis quantification. SARS-CoV-2 genomic RNA (forward primer 5'-GTGARATGGTCATGTGTGGCGG-3', SEQ ID NO: 28 reverse primer 5'-CARATGTTAAASACACTATTAGCATA-3', SEQ ID NO: 29 and MGB probe 1 5'-CAGGTGGAACCTCATCAGGAGATGC-3' SEQ ID NO: 30) and SARS-CoV-2 subgenomic messenger RNA (sgmRNA) N (forward primer 5'-CCAACCAACTTTCGATCTCTTGT-3', SEQ ID NO: 31 reverse primer 5-'GGGTGCATTTCGCTGATTTT-3', SEQ ID NO: 32 and MGB probe 2 5'-TTCTCTAAACGAACAAACTA-3' SEQ ID NO: 33) custom probes were designed for this analysis; forward and reverse primers were purchased from Sigma-Aldrich, and MGB probes were purchased from Eurofins Genomics. Data were acquired with a QuantStudio 5 Real-Time PCR system (Applied Biosystems) and analyzed with ABI PRISM 7500 software, version 2.0.5. The relative quantifications were performed using the cycle threshold (2-ΔΔCT) method. To normalize differences in RNA sampling, the expression of mouse 18S ribosomal RNA was analyzed using a specific TaqMan Gene Expression Assay (Mm03928990_g1; ThermoFisher Scientific).

### 5.5 Lung Histopathology

Mice were euthanized at the indicated day postinfection (dpi) or day postchallenge (dpc). The left lungs of infected mice were fixed in 10% zinc formalin for 24 h, at 4 °C and paraffin embedded. Serial longitudinal 5-µm sections were stained with hematoxylin and eosin by the Histology Service at CNB-CSIC (Madrid, Spain) and subjected to histopathological examination with a ZEISS Axiophot fluorescence microscope. Samples were obtained using a systematic uniform random procedure, consisting of serial parallel slices made at a constant thickness interval of 50 µm. Histopathology analysis was conducted in a blind manner by acquiring images of 50 random microscopy fields from around 40 nonadjacent sections for each of the three independent mice analyzed per treatment group.

No significant pathological changes were observed in the lungs of mice infected with SARS-CoV-2-Δ[3,E,6,7] or SARS-CoV-2-Δ[3,E,7,8] at 3 dpi. In contrast, the lungs of mice infected with SARS-CoV-2-WT virus showed clear alveolar wall thickening and peribronchial cuffing. By 6 dpi, examination of lungs of SARS-CoV-2-infected mice revealed generalized infiltration and parenchyma consolidation, as well as edema in the airspaces, whereas the lungs of mice infected with SARS-CoV-2-Δ[3,E,6,7] or SARS-CoV-2-Δ[3,E,7,8] replicon remained similar to those of uninfected mice.

### 6) HUMORAL AND CELLULAR RESPONSE IN IMMUNIZED MICE ELICITED BY SARS-CoV-2 RNA REPLICON DELETION MUTANTS.

### 6.1 Indirect Enzyme-Linked ImmunoSorbent Assay (ELISA)

Serum samples from mice immunized with SARS-CoV-2 Δ[3,E], SARS-CoV-2 Δ[3,E,7,8] or SARS-CoV-2 Δ[3,E,6,7] or mock-immunized were collected at 21 days post-immunization (dpi). Samples were diluted to 1:100. Spike protein-specific IgG antibody levels and RBD-specific (receptor binding domain-specific) IgG antibody levels were measured by Indirect Enzyme-Linked ImmunoSorbent Assay (ELISA).

96-well Nunc Maxisorp immune plates (Thermo Scientific) were coated with 100 ng/well of SARS-CoV-2 RBD protein. The protein was diluted in PBS at a final concentration of 2 µg/ml in 50 µl per well. The plates were incubated at 4 °C for 24 hours.

Unbound protein was removed, ELISA plates were washed three times with PBS-0.05% Tween20 (PBST) and 200 µl of PBST-3% milk (blocking buffer) were added.

Plates were incubated for 1.5 hours at room temperature. Meanwhile, serum samples were inactivated at 56 °C for 30 minutes and serial dilutions were prepared in PBST-1% milk. 50 µl of diluted sera were added to the wells and the plates were incubated for 2 hours at room temperature. Plates were washed with PBST, secondary HRP-conjugated goat anti-mouse IgG and IgA (Southern Biotech), depending on the evaluated antibody isotype, were diluted in PBST-1% milk according to manufacturer instructions and 50 µl/well were added. After 1 hour incubation at room temperature, plates were washed and 50 µl of supersensitive TMB were added. The plates were incubated at room temperature in darkness approximately 5-10 minutes and the reaction was stopped with 0.5 M H₂SO₄. Optical density was read at 450 nm with a ELISA plate reader (Tecan).

Results are shown in **Figure 12** and illustrate high levels of anti-spike and anti-RBD IgG were detected in immunized mice (21 dpi) for all tested replicons. The specific response against SARS-CoV-2 detected in the serum, strongly demonstrates that the SARS-CoV-2-Δ[3,E], SARS-CoV-2-Δ[3,E,7,8], SARS-CoV-2-Δ[3,E,6,7] polynucleotides will provide immunity against subsequent viral infection.

### 6.2. Plaque Reduction Neutralization test (PRNT50)

Vero-E6 cells were seeded in 24-well tissue culture plates 24 hours prior to neutralization. The serum samples from mice immunized with SARS-CoV-2 Δ[3,E], SARS-CoV-2 Δ[3,E,7,8] or SARS-CoV-2 Δ[3,E,6,7] or from non-immunized mice were inactivated 56 °C for 30 minutes, serial dilutions of these serum samples were prepared with 2% FBS DMEM and incubated for 1 hour at 37 °C with 50 PFU of SARS-CoV-2 in 1:1 volume proportion. The mixture serum:virus was added to the pre-seeded 24-well plates and were incubated for 1 hour at 37 °C. The overlay medium (2x DMEM with 1% agarose) was prepared and added to the plates, which were incubated for 3 days in 5% CO₂ 37 °C incubator. The cells were fixed with 10% formaldehyde solution and stained with crystal violet.

Neutralizing antibodies were measured by 50% plaque reduction neutralizing test (PRNT50), considering the neutralizing antibody titer as the highest serum dilution that reduce 50% the number of plaques in comparison to the plaques formed by the only virus. Neutralizing antibodies against SARS-CoV-2 WU-WT were not detected in non-immunized groups, whereas serum samples from immunized mice at 21 dpi neutralized SARS-CoV-2 WU-WT with a neutralization capacity (TCID₅₀) of > 8 (**Figure 13**).

### 6.3. Cellular Immune Response in Mice Immunized with SARS-CoV-2 RNA Polynucleotides

To assess whether the SARS-CoV-2 Δ[3,E], SARS-CoV-2 Δ[3,E,7,8], SARS-CoV-2 Δ[3,E,6,7] replicons are able to elicit a cellular immune response, K18-hACE2 mice were intranasally immunized with the SARS-CoV-2 replicons and sacrificed after 15 days. Lungs of mice were flushed with RPMI medium supplemented with HEPES, β-mercaptoethanol, and L-glutamine. Cellular debris were removed by centrifugation and lung samples stored at -20°C.

The immune responses of CD-4+ and CD-8+ T-cells were analyzed by ICS as previously described (Garcia-Arriaza et al, 2021; Pérez et al., 2022) with some modifications. Collected cells were seeded on plates and stimulated for 24 hours in RPMI 10% FBS and the SARS-CoV-2 S1 and S2 peptide pools (1mg/ml). Cells were washed and stained for analysis. Specific fluorochrome-conjugated antibodies were used for functional analysis (CD3-phycoerythrin (PE)-CF594, CD4-allophycocyanin (APC)-Cy7, CD8-V500, IFN-g-PE-Cy7, TNF-a-PE, and IL-2-APC) and phenotypic analysis (CD62L-Alexa Fluor 700 and CD127-peridinin chlorophyll protein (PerCP)-Cy5.5). Cell analysis was conducted by flow cytometry. Data analysis was performed with the FlowJo software.

Immune response of T-cells in bronchoalveolar lavages of immunized mice was evaluated by analyzing the percentages of CD-4+ or CD-8+ T-cells with double expression of tumor necrosis factor alpha (TNFα) and interferon gamma (IFNγ) cytokines. The results showed high percentages of CD-8+ T-cells expressing TNFα and IFNy in the samples of mice immunized with the different replicons, as compared to mock, and high percentages of double positive cells were only shown when samples were stimulated with the SARS-CoV-2 peptide pool (S1 and S2). Percentages of CD-8+ cells with expression of TNFα and IFNy in SMN-stimulated samples of mice immunized with SARS-CoV-2 replicons were: SARS-CoV-2 Δ[3,E] 41.2%, SARS-CoV-2 Δ[3,E,6,7] 52.5% and SARS-CoV-2 Δ[3,E,7,8] 21.4% (**Figure 14**).

### 7) PROTECTION ELICITED BY SARS-CoV-2-[3,E], -Δ[3,E,6,7], and Δ[3,E,7,8] POLYNUCLEOTIDES IN VIVO

The same protocols described in Example 3, above, was used in this Example. 16- to 24-week-old female mice were anesthetized with isoflurane and intranasally inoculated with 50 µL of virus diluted in DMEM. SARS-CoV-2 and its deletion derived replicons were evaluated using 10,000 PFU of the indicated virus per mouse to assess virulence and 100,000 FFU of virulent SARS-CoV virus in challenge experiments. Weight loss and mortality were evaluated daily. To determine viral titers, lungs were homogenized in 2 mL of PBS containing 100 IU/mL penicillin, 0.1 mg/mL streptomycin, 50 µg/mL gentamicin, and 0.5 µg/mL amphotericin B (Fungizone) using a gentleMACS dissociator (Miltenyi Biotec, Inc.). Virus titrations were performed in VeroE6 or VeroE6-TMPRSS2 cells as described above. Viral titers were expressed as PFU counts per gram of tissue. All work with infected animals was performed in an Animal Biosafety Level 3+ laboratory wearing personal protection equipment (3M).

K18-hACE2 mice (23-week-old) were immunized by administrating the RNA replicons SARS-CoV-2 Δ[3,E], SARS-CoV-2 Δ[3,E,7,8], SARS-CoV-2 Δ[3,E,6,7] as VLPs or mock immunized. After three weeks post-immunization, mice were challenged with a lethal dose of SARS-CoV-2 WT (10⁵ PFU/animal).

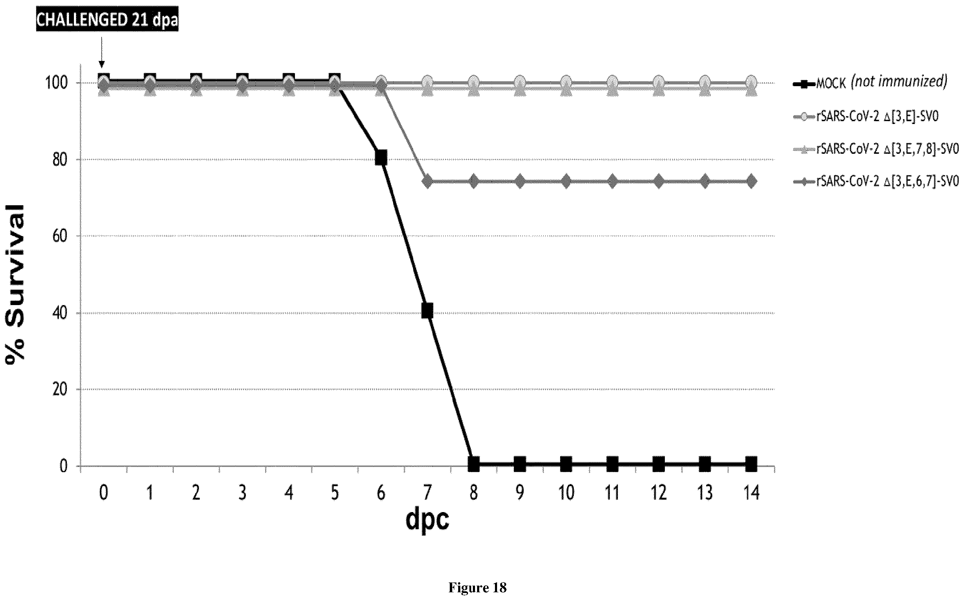

Body weight and survival rate of infected mice were monitored for 14 days. Animals suffering weight losses as much as 20% of the initial weight were sacrificed according to the established end point criteria. In contrast to mock-immunized mice, immunized mice started to recover normal body weight at day 6 post-infection and reached a body weight of > 90 % of initial body weight. Survival rates were additionally monitored showing protection of mice when immunized with one of the three replicons. Non-immunized mice lost weight and died between 6 and 8 dpc (**Figure 17** **and** **Figure 18**). However, mice immunized with SARS-CoV-2 Δ[3,E,6,7] showed 75 % protection and mice immunized with SARS-CoV-2 Δ[3,E] or SARS-CoV-2 Δ[3,E,7,8] showed 100 % protection.

These results demonstrated that SARS-CoV-2 Δ[3,E], SARS-CoV-2 Δ[3,E,7,8] and SARS-CoV-2 Δ[3,E,6,7] replicons induced high protection levels in K18-hACE2 mice against a lethal dose of SARS-CoV-2 WT virus. The results indicate the SARS-CoV-2 Δ[3,E,7,8] replicon showing 100 % protection as a promising candidate for vaccine development.

### SEQUENCE LISTING

SEQ ID NO: 1: SARS-CoV-2 without amendments Genbank: MN908947.3 47
SEQ ID NO: 2: V0; SARS-CoV-2-Δ[3,E,6,7,8]
SEQ ID NO: 3: V1; SARS-CoV-2- nsp1ΔD-Δ[,3,E,6,7,8]
SEQ ID NO: 4: S gene polynucleotide sequence without amendments
SEQ ID NO: 5: SARS-CoV-2 S gene polynucleotide sequence codon-optimized for human codon usage
SEQ ID NO: 6: protein 3a amino acid sequence GenBank YP_009724391
SEQ ID NO: 7: protein E amino acid sequence GenBank YP_009724392
SEQ ID NO: 8: pBAC-SARS-CoV-2-FL: pBAC sequence (nucleotides 1 to 7889), SARS- CoV-2 genome sequence (nucleotides 7890 to 37784) and pBAC sequence (nucleotides 37785 to 38125), including genetic markers in the form of silent mutations
SEQ ID NO: 9: F1 fragment SARS-CoV-2 polynucleotide sequence
SEQ ID NO: 10: F2 fragment SARS-CoV-2 polynucleotide sequence
SEQ ID NO: 11: F3 fragment SARS-CoV-2 polynucleotide sequence
SEQ ID NO: 12: F4 fragment SARS-CoV-2 polynucleotide sequence
SEQ ID NO: 13: F5 fragment SARS-CoV-2 polynucleotide sequence
SEQ ID NO: 14: F6 fragment SARS-CoV-2 polynucleotide sequence
SEQ ID NO: 15: SARSCoV2-Δ3 polynucleotide sequence
SEQ ID NO: 16: SARSCoV2-ΔE polynucleotide sequence
SEQ ID NO: 17: SARSCoV2-Δ[3,E] polynucleotide sequence
SEQ ID NO: 18: SARS-CoV-2-Δ6 polynucleotide sequence, containing two point substitutions (c.27041A>C and c.27044A>C) in comparison to SEQ ID NO: 1.
SEQ ID NO: 19: SARS-CoV-2-Δ7 polynucleotide sequence, wherein 7a and 7b genes were deleted.
SEQ ID NO: 20: SARS-CoV-2-Δ7a polynucleotide sequence
SEQ ID NO: 21: SARS-CoV-2-Δ7b polynucleotide sequence
SEQ ID NO: 22: SARS-CoV-2-Δ8 polynucleotide sequence
SEQ ID NO: 23: SARS-CoV-2-Δ[6,8] polynucleotide sequence, containing two point substitutions (c.27041A>C and c.27044A>C) in comparison to SEQ ID NO: 1
SEQ ID NO: 24: SARS-CoV-2-Δ[6,7,8] polynucleotide sequence, containing two point substitutions (c.27041AOC and c.27044A◊C) in comparison to SEQ ID NO: 1
SEQ ID NO: 25: Fnsp1-ΔD polynucleotide sequence: pUC57 sequence (nucleotides 1 to 42) and SARS-CoV-2 nsp1-ΔD sequence (nucleotides 43-836)
SEQ ID NO: 26: WH-25155-VS
SEQ ID NO: 27: WH-28957-RS
SEQ ID NO: 28: SARS-CoV-2 genomic RNA forward primer
SEQ ID NO: 29: SARS-CoV-2 genomic RNA reverse primer
SEQ ID NO: 30: MGB probe 1
SEQ ID NO: 31: subgenomic messenger RNA (sgmRNA) N forward primer
SEQ ID NO: 32: subgenomic messenger RNA (sgmRNA) N reverse primer
SEQ ID NO: 33: MGB probe 2
SEQ ID NO: 34: SARS-CoV-2 S protein amino acid sequence, encoded by SEQ ID NO: 4
SEQ ID NO: 35: SARS-CoV-2 S protein amino acid sequence, encoded by SEQ ID NO: 5
SEQ ID NO: 36: SARS-CoV-2 ORF8 gene sequence
SEQ ID NO: 37: SARS-CoV-2 ORF6 gene sequence
SEQ ID NO: 38: SARS-CoV-2 ORF7a gene sequence
SEQ ID NO: 39: SARS-CoV-2 ORF7b gene sequence
SEQ ID NO: 40: F4 fragment SARS-CoV-2 polynucleotide sequence of Delta variant
SEQ ID NO: 41: F4 fragment SARS-CoV-2 polynucleotide sequence of Omicron variant
SEQ ID NO: 42: SARS-CoV-2-Δ[3,E,6,7] polynucleotide sequence
SEQ ID NO: 43: SARS-CoV-2-Δ[3,E,7,8] polynucleotide sequence
SEQ ID NO: 44: SARS-CoV-2-Δ[3,E,6,8] polynucleotide sequence
SEQ ID NO: 45: Fnsp1_ΔA polynucleotide sequence
SEQ ID NO: 46: Fnsp1_Δ[B,D] polynucleotide sequence
SEQ ID NO: 47: SARS-CoV-2-Δ[3,E,6] polynucleotide sequence, containing two point substitutions (c.27041A>C and c.27044A>C) in comparison to SEQ ID NO: 1
SEQ ID NO: 48: SARS-CoV-2-Δ[3,E,7] polynucleotide sequence
SEQ ID NO: 49: SARS-CoV-2-Δ[3,E,8] polynucleotide sequence
SEQ ID NO: 50: SARS-CoV-2-Δ[3,E,6,8] polynucleotide sequence, containing two point substitutions (c.27041A>C and c.27044A>C) in comparison to SEQ ID NO: 1

### REFERENCES

Zhang X, Liu Y, Liu J, Bailey AL, Plante KS, Plante JA, Zou J, Xia H, Bopp NE, Aguilar PV, Ren P, Menachery VD, Diamond MS, Weaver SC, Xie X, Shi PY. A trans-complementation system for SARS-CoV-2 recapitulates authentic viral replication without virulence. Cell. 2021 Apr 15;184(8):2229-2238.e13. doi: 10.1016/j.cell.2021.02.044. Epub 2021 Feb 23. PMID: 33691138; PMCID: PMC7901297.
Silvas JA, Vasquez DM, Park JG, Chiem K, Allué-Guardia A, Garcia-Vilanova A, Platt RN, Miorin L, Kehrer T, Cupic A, Gonzalez-Reiche AS, Bakel HV, Garcia-Sastre A, Anderson T, Torrelles JB, Ye C, Martinez-Sobrido L. Contribution of SARS-CoV-2 Accessory Proteins to Viral Pathogenicity in K18 Human ACE2 Transgenic Mice. J Virol. 2021 Aug 10;95(17):e0040221. doi: 10.1128/JVI.00402-21. Epub 2021 Aug 10. PMID: 34133899; PMCID: PMC8354228.
Dediego ML, Pewe L, Alvarez E, Rejas MT, Perlman S, Enjuanes L. Pathogenicity of severe acute respiratory coronavirus deletion mutants in hACE-2 transgenic mice. Virology. 2008 Jul 5;376(2):379-89. doi: 10.1016/j.virol.2008.03.005. Epub 2008 May 2. PMID: 18452964; PMCID: PMC2810402.
Almazán F, DeDiego ML, Sola I, Zuñiga S, Nieto-Torres JL, Marquez-Jurado S, Andrés G, Enjuanes L. Engineering a replication-competent, propagation-defective Middle East respiratory syndrome coronavirus as a vaccine candidate. mBio. 2013 Sep 10;4(5):e00650-13. doi: 10.1128/mBio.00650-13. PMID: 24023385; PMCID: PMC3774192.
Almazán F, Márquez-Jurado S, Nogales A, Enjuanes L. Engineering infectious cDNAs of coronavirus as bacterial artificial chromosomes. Methods Mol Biol. 2015;1282:135-52. doi: 10.1007/978-1-4939-2438-7_13. PMID: 25720478; PMCID: PMC4726977.
Mairhofer J, Grabherr R. Rational vector design for efficient non-viral gene delivery: challenges facing the use of plasmid DNA. Mol Biotechnol. 2008 Jun;39(2):97-104. doi: 10.1007/s12033-008-9046-7. PMID: 18327557.
Mairhofer J, Pfaffenzeller I, Merz D, Grabherr R. A novel antibiotic free plasmid selection system: advances in safe and efficient DNA therapy. Biotechnol J. 2008 Jan;3(1):83-9. doi: 10.1002/biot.200700141. PMID: 17806101.
Almazan, F., Gonzalez, J.M., Penzes, Z., Izeta, A., Calvo, E., Plana-Duran, J., Enjuanes, L., 2000. Engineering the largest RNA virus genome as an infectious bacterial artificial chromosome. Proc. Natl. Acad. Sci. USA 97, 5516-5521**.**
Almazan, F., Sola, I., Zuñiga, S., Marquez-Jurado, S., Morales, L., Becares, M., Enjuanes, L., 2014. Coronavirus reverse genetic systems: Infectious clones and replicons. Virus Res. 189, 262-270.
Gutierrez-Alvarez J. et al, 2021. Middle East respiratory syndrome coronavirus vaccine based on a propagation-defective RNA replicon elicited sterilizing immunity in mice. Proc. Natl. Acad. Sci. USA 118 : e2111075118.

## Claims

1. A polynucleotide comprising:
(a) a polynucleotide sequence of SEQ ID NO: 43 (SARS-CoV-2Δ[3, E, 7 and 8] mutant) or SEQ ID NO: 42 (SARS-CoV-2Δ[3, E, 6 and 7] mutant); or
(b) a variant of SEQ ID NO: 43 or a variant of SEQ ID NO: 42, wherein the variant has at least 96 % identity, preferably at least 97 % or at least 98 % or at least 99 % identity to SEQ ID NO: 43 or SEQ ID NO: 42.

2. The polynucleotide according to claim 1, wherein the variant polynucleotide sequence is further **characterized in that**:
(b1) the variant encodes a replication-competent mutant of the SARS-CoV-2 RNA genome, wherein replication-competent means that the mutant SARS-CoV-2 RNA genome can produce copies upon transfection of VeroE6-TMPRSS2 cells; and
(b2) the variant encodes a propagation-defective mutant of the SARS-CoV-2 RNA genome, wherein propagation-defective means that upon transfection of VeroE6-TMPRSS2 cells the mutant SARS-CoV-2 RNA genome is not able to assemble a virus or a VLP on the basis of the proteins encoded in the variant polynucleotide, which virus or VLP would be capable of infecting other VeroE6-TMPRSS2 cells.

3. The polynucleotide according to claim 1 or 2, wherein the variant polynucleotide sequence is further **characterized in that**:
(c) upon infection or transfection of a VeroE6-TMPRSS2 cell culture the mutant SARS-CoV-2 RNA genome is able to produce a viral titre of at least 10³ FFU/ml or a viral titre of at least 10⁴ FFU/ml or at least 10⁵ FFU/ml or at least 10⁶ FFU/ml.

4. The polynucleotide according to one of claims 1 to 3, wherein the variant polynucleotide:
(d1) does not comprise a sequence encoding a protein corresponding to the proteins of ORF 3a, ORF E, ORF 6 and ORF 7; or
(d2) does not comprise a sequence encoding a protein corresponding to the proteins of ORF 3a, ORF E, ORF 7 and ORF 8.

5. The polynucleotide according to one of claims 1 to 4, wherein SEQ ID NO: 43, SEQ ID NO: 42 or the respective the variant polynucleotide:
(e) comprises a deletion of at least 18 consecutive nucleotides in the sequence encoding a full-length nsp1 domain consisting of nucleotides 266 to 805 of SEQ ID NO: 43 or SEQ ID NO: 42 ; and/or
(f) comprises a deletion of at least 6 consecutive nucleotides in the sequence encoding a furin cleavage site consisting of nucleotides 23603 to 23617 of SEQ ID NO: 43 or SEQ ID NO: 42.

6. The polynucleotide according to one of claims 1 to 5, wherein the polynucleotide is RNA or DNA.

7. A Virus Like Particle (VLP) comprising a polynucleotide according to one of claims 1 to 6.

8. An expression vector comprising a DNA sequence comprising a polynucleotide of one of claims 1 to 5.

9. A cell transfected with polynucleotide according to one of claims 1 to 6, infected with the VLP according to claim 7 and/or transfected with the expression vector according to claim 8.

10. A cell according to claim 8, wherein the cell is selected from cell lines VeroE6-TMPRSS2, Vero E6 or BHK21.

11. A method of producing a VLP comprising the steps of:
(a) transfecting a cell with an expression vector according to claim 8, wherein the cell expresses proteins encoded in SEQ ID NO: 6 and SEQ ID NO: 7 amino acid residues sequences; and
(b) purifying the VLPs from the supernatant.

12. A vaccine composition comprising a VLP according to claim 7 or a polynucleotide according to one of claims 1 to 6, and optionally further comprising:
- at least one pharmaceutically acceptable excipient and/or
- at least one chemical or biological adjuvant or immunostimulant.

13. The vaccine composition according to claim 12, for use in protecting a patient against SARS-CoV-2 infection.

14. The vaccine composition according to one of claims 12 or 13, wherein the composition is formulated for topical, intranasal, oral, subcutaneous or intramuscular administration, wherein the vaccine is preferably administered using a dosage regimen comprising a first administration at day 1 and at least one further administration at least 21 days later.

15. The vaccine composition according to one of claims 12 to 14, wherein the vaccine comprises two different polynucleotides according to one of claims 1 to 6, wherein:
(a) one polynucleotide comprises the gene coding for protein S of the Delta strain comprising nucleotides 1479 to 5228 of SEQ_ID 40 or variant thereof having at least 96 % identity, preferably at least 97 % or at least 98 % or at least 99 % identity to nucleotides 1479 to 5228 of SEQ ID NO: 40; and
(b) the other polynucleotide comprises the gene coding for protein S of the Omicron strain comprising nucleotides 1479 to 5216 of SEQ_ID 41 or variant thereof having at least 96 % identity, preferably at least 97 % or at least 98 % or at least 99 % identity to nucleotides 1479 to 5216 of SEQ ID NO: 41.
